(19) Europäisches Patentamt
European Patent Office
Office européen des brevets

(11) Veröffentlichungsnummer : **0 347 694 B1**

(12) # EUROPÄISCHE PATENTSCHRIFT

(45) Veröffentlichungstag der Patentschrift : **26.08.92 Patentblatt 92/35**

(51) Int. Cl.$^5$ : **C07C 69/743,** C07C 255/39, C07C 33/18, A01N 53/00

(21) Anmeldenummer : **89110562.9**

(22) Anmeldetag : **10.06.89**

(54) **Pyrethroide, Verfahren zu ihrer Herstellung und ihre Verwendung zur Bekämpfung von Schädlingen.**

(30) Priorität : **21.06.88 DE 3820895**

(43) Veröffentlichungstag der Anmeldung : **27.12.89 Patentblatt 89/52**

(45) Bekanntmachung des Hinweises auf die Patenterteilung : **26.08.92 Patentblatt 92/35**

(84) Benannte Vertragsstaaten : **AT BE CH DE ES FR GB GR IT LI NL SE**

(56) Entgegenhaltungen :
**EP-A- 0 031 199**
**DE-A- 1 542 985**
**FR-A- 2 531 074**
**US-A- 4 332 815**

(73) Patentinhaber : **BASF Aktiengesellschaft**
**Carl-Bosch-Strasse 38**
**W-6700 Ludwigshafen (DE)**

(72) Erfinder : **Wolf, Bernd, Dr.**
**Eichenstrasse 11**
**W-6704 Mutterstadt (DE)**
Erfinder : **Theobald, Hans, Dr.**
**Queichstrasse 6**
**W-6703 Limburgerhof (DE)**
Erfinder : **Zombik, Winfried, Dr.**
**Kallstadter Strasse 4**
**W-6804 Ilvesheim (DE)**
Erfinder : **Goetz, Norbert, Dr.**
**Schoefferstrasse 25**
**W-6520 Worms 1 (DE)**
Erfinder : **Wild, Jochen, Dr.**
**An der Marlach 7**
**W-6705 Deidesheim (DE)**
Erfinder : **Harreus, Albraecht, Dr.**
**Teichgasse 13**
**W-6700 Ludwigshafen (DE)**
Erfinder : **Hofmeister, Peter, Dr.**
**Bernard-Humblot-Strasse 12**
**W-6730 Neustadt (DE)**
Erfinder : **Kuenast, Christoph, Dr.**
**Salierstrasse 2**
**W-6701 Otterstadt (DE)**
Erfinder : **DeKramer, Jacobus Jan, Dr.**
**Kalmitweg 53**
**6703 Limburgerhof (DE)**

EP 0 347 694 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Benzylester, Verfahren zu ihrer Herstellung, Schädlingsbekämpfungsmittel, die diese Ester als Wirkstoffe enthalten, sowie Verfahren zur Bekämpfung von Schädlingen mit diesen Wirkstoffen

Es ist bereits bekannt, daß bestimmte Ester des α,m-Dimethylbenzylalkohols insektizide Eigenschaften aufweisen [Pestic. Sci. 1 (2), 49-52 (1970); Pestic. Sci. 1 (5), 220-223 (1970); Pestic. Sci. 3 (1), 25-28 (1972); US Patent 4 332 815]. Unter anderem sind aus der Literatur bereits folgende Vertreter dieser Verbindungsklasse bekannt: im Benzyl-Teil durch Halogen und/oder C-organische Reste substituierte Pyrethroide (DE-A 15,42,985); im Benzyl-Teil mehrfach durch Fluor und einfach durch C-organische Reste substituierte Pyrethroide (EP-A 31,199); gegebenenfalls im Aryl-Teil substituierte Benzyl- und Heteroarylmethylester (US-A 4,332,815). Weiterhin sind aus FR 2531 074 bzw. Pestic. Sci. 37 (6), 691-700 (1986) einige Ester von α-Methylbenzylalkoholen, die in m-stellung einen gegebenenfalls endständig substituierten Allylrest tragen, bekannt. Die insektizide Wirkung dieser Ester ist jedoch unter bestimmten Bedingungen (z.B. bei niedrigen-Aufwandkonzentrationen) in den meisten Fällen unbefriedigend. In J. Agric Food Chem 29 1118-1122 (1981) wird berichtet, daß der 3-(2′,2′-Dichlorvinyl)-2,2-dimethylcyclopropancarbonsäure-(3-isopropyl-benzyl)ester keine insektizide Aktivität aufweist.

Es wurde nun gefunden, daß Benzylester der allgemeinen Formel I

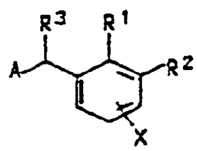

(I)

in der

$R^1$ Methyl, Ethyl, Halogen, Methoxy oder Ethoxy,

$R^2$ Alkyl, Alkenyl, Cycloalkyl, Cycloalkenyl, Bicycloalkyl, Bicycloalkenyl, $C_1$-$C_5$-alkylsubstituiertes Cycloalkyl, $C_1$-$C_5$-alkylsubstituiertes Cycloalkenyl, $C_1$-$C_5$-alkylsubstituiertes Bicycloalkyl oder $C_1$-$C_5$-alkylsubstituiertes Bicycloalkenyl,

$R^3$ Wasserstoff, Cyano, $C_2$-$C_4$-Alkinyl, $C_2$-$C_4$-Alkanyl oder $C_1$-$C_4$-Alkyl.

A den Carboxylat-Rest einer bei Pyrethroiden typischen Säurekomponente und

X Wasserstoff oder Halogen bedeuten, mit der Maßgabe, daß $R^2$ nicht den Rest $CH_2$-CH=CH-B bedeutet, wenn B für Wasserstoff, Alkyl oder Alkenyl steht und zugleich $R^1$ die Bedeutung Methyl oder Halogen hat, sowie ferner mit der Maßgabe, daß $R^2$ nicht Methyl bedeutet, wenn zugleich $R^1$ für Methyl steht, starke insektizide und akarizide Wirkung aufweisen.

In der vorliegenden Anmeldung haben, falls nicht anders angegeben, die folgenden Bezeichnungen die folgende Bedeutung:

"Alkyl" bezieht sich auf eine gerade oder verzweigte Alkylgruppe mit 1 bis 20, insbesondere 1 bis 12 Kohlenstoffatomen. Beispielsweise seien Methyl, Ethyl, Propyl, Isopropyl, N-Butyl, iso-Butyl, sec.-Butyl und tert.-Butyl genannt.

"Alkenyl" bedeutet eine gerade oder verzweigte, ethylenisch ungesättigte Kohlenwasserstoffgruppe mit 2 bis 20 Kohlenstoffatomen und 1 bis 10 ethylenischen Bindungen, insbesondere mit 2 bis 12 Kohlenstoffatomen und 1 bis 5 ethylenischen Bindungen. Beispielsweise seien Vinyl, Isopropenyl, 1-Butenyl, 1,5-Hexadienyl, 1-Methyl-propenyl und 1-Ethyl-vinyl genannt.

Die Bezeichnung "Cycloalkyl" bedeutet eine Cycloalkylgruppe mit 3 bis 8, insbesondere 3 bis 6 C-Atomen, im Ring wie Cyclopropyl, Cyclobutyl, Cyclopentyl oder Cyclohexyl. Diese Cycloalkylgruppe ist unsubstituiert oder substituiert durch ein oder mehrere, z.B. 1 bis 4 verzweigte oder unverzweigte $C_1$-$C_5$-Alkylreste wie Methyl, Ethyl, Propyl, Isopropyl, Butyl, sec-Butyl, tert-Butyl oder Pentyl. Beispielsweise seien 3,5-Diethylcyclohexyl und Tetramethylcyclopropyl genannt.

"Cycloalkenyl" bezieht sich auf eine Cycloalkenylgruppe mit 3 bis 8, insbesondere 3 bis 6 C-Atomen, im Ring Cyclopropenyl, Cyclobutenyl, Cyclopentenyl, Cyclopentadienyl oder Cyclohexadienyl. Diese Cycloalkenylgruppe ist unsubstituiert oder substituiert durch ein oder mehrere z.B. 1 bis 4 verzweigte oder unverzweigte $C_1$-$C_5$-Alkylreste wie Methyl, Ethyl, Propyl, Isopropyl, Butyl, sec-Butyl, tert-Butyl oder Pentyl. Beispielsweise seien 1-Cyclopentenyl, 1-Cyclohexenyl, 3,5-Dimethyl-1-cyclohexenyl und 1,3-Cyclohexadienyl genannt.

"Bicycloalkyl" bedeutet eine Bicycloalkylgruppe mit 5 bis 12, insbesondere 6 bis 8 Kohlenstoffatomen im Bicyclus wie z.B. 2-Norbornyl oder Bicyclo[4.1.0]hept-1-yl. Dieser Bicyclus ist unsubstituiert oder substituiert durch ein oder mehrere, z.B. 1 bis 2 verzweigte oder unverzweigte Alkylreste wie Methyl, Ethyl, Propyl, Isopropyl, Butyl, sec-Butyl oder Pentyl. Beispielsweise sei 2,6-Dimethyl-bicyclo[4.1.0]hept-1-yl genannt.

"Bicycloalkenyl" steht für eine ungesättigte Bicycloalkylgruppe mit 5 bis 12, insbesondere 6 bis 8 Kohlenstoffatomen im Bicyclus und einer oder mehreren z.B. 1 oder 2 ethylenischen Doppelbindungen, wie z.B. Norbornen-2-yl, Norbornadien-2-yl. Diese Bicycloalkenylgruppe ist unsubstituiert oder substituiert durch 1 oder mehrere, z.B. 1 bis 3 verzweigte oder unverzweigte $C_1$-$C_5$-Alkylreste wie Methyl, Ethyl, Propyl, Isopropyl, Butyl, sec-Butyl oder Pentyl. Beispielsweise sei 7,7-Dimethylbicyclo[4.1.0]hept-2-en-1-yl genannt.

Bevorzugt stehen in diesen Verbindungen für

$R^1$ Methyl, Brom, Chlor oder Fluor,

$R^2$ verzweigte Alkyl- oder Alkenylreste mit 3 bis 8 Kohlenstoffatomen wie Isopropyl, Isopropenyl, sec-Butyl, tert-Butyl, 1-But-en-2-yl, 2-Butenyl, 1,3-Butadienyl, iso-Pentyl, sec-Pentyl, 3-Penten-2-yl, 1-Penten-2-yl oder sec-Hexyl, wobei die drei erstgenannten Reste besonders bevorzugt sind, $C_3$-$C_8$-Cycloalkyl oder Cycloalkenyl, wie Cyclopentyl, 1-Cyclopentenyl, Cyclohexyl, 1-Cyclohexenyl, insbesondere Cyclopropyl; $C_6$-$C_8$-Bicycloalkyl oder Bicycloalkenyl wie 2-Norbornyl, 2-Norbornen-2-yl oder 2,5-Norbornadien-2-yl,

$R^3$ Wasserstoff, Ethinyl oder Cyano,

X Wasserstoff oder Fluor

und

A die Carboxylatreste II, III oder IV

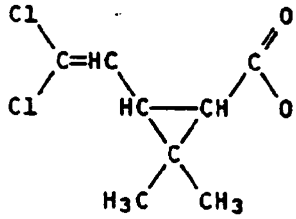

(II)

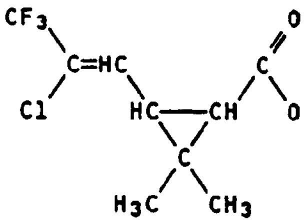

(III)

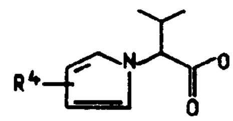

(IV)

wobei

$R^4$ die Bedeutung Wasserstoff, $C_1$-$C_4$-Alkyl oder $C_1$-$C_4$-Halogenalkyl wie Methyl, Ethyl, Propyl, Isopropyl, Butyl, iso-Butyl, sec-Butyl, tert-Butyl, Trifluormethyl, Trichlormethyl oder 2,2,2-Trifluorethyl hat.

Die Ester der allgemeinen Formel I können durch Umsetzung der Säuren A-H oder Derivaten dieser Säuren wie Säurechloriden, Säureanhydriden o.ä. mit Benzylalkoholen der allgemeinen Formel V oder Derivaten von V nach folgendem Reaktionsschema gewonnen werden.

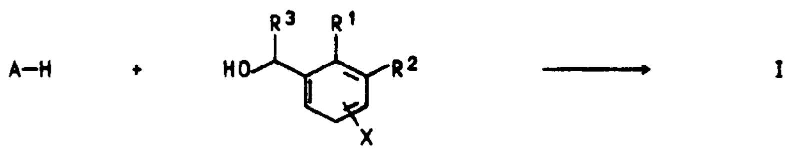

(Die Reste $R^1$ bis $R^3$, X und A haben die in Anspruch 1 genannte Bedeutung).

Die Umsetzung kann in an sich bekannter Weise durch Zusatz von Katalysatoren wie Schwefelsäure, Halogenwasserstoff, Sulfonsäuren, sauren Ionenaustauschern beschleunigt und das Veresterungsgleichgewicht im gewünschten Sinne verschoben werden, indem man dem Reaktionsgemisch das Wasser oder den Ester I entzieht, z.B. durch azeotrope Destillation oder durch Bindung des Wassers an Schwefel- oder Halogenwasserstoffsäure.

Ebensogut können die entsprechenden Säurechloride mit den Alkoholen der Formel V in Gegenwart eines Säureakzeptors umgesetzt werden (vgl. Houben-Weyl, Methoden der organ. Chemie, Band VIII, S. 541 ff, Georg-Thieme-Verlag, Stuttgart 1952).

Als Säurebindemittel eignen sich die üblichen basischen Mittel, insbesondere aliphatische, aromatische und heterocyclische Amine, z.B. Triethylamin, Dimethylamin, Piperidin, Dimethylanilin, Dimethylbenzylamin, Pyridin und 2-Picolin.

Die Umsetzung kann in einem Lösungs- oder Verdünnungsmittel ausgeführt werden. Hierzu sind die angeführten Säureakzeptoren selbst oder beispielsweise folgende Lösungs- oder Verdünnungsmittel oder Gemische derselben geeignet:

Aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlorethan, Chlorbenzol; Ether wie

Diethyl- und Di-n-butylether, Methyl-tert.-butylether, Tetrahydrofuran, Dioxan; Ketone, beispielsweise Aceton, Methylethylketon, Methylisopropylketon; ferner Nitrile wie Acetonitril und Propionitril.

Üblicherweise setzt man die Ausgangsstoffe in stöchiometrischem Verhältnis ein. Ein Überschuß des einen oder anderen kann in Einzelfällen aber durchaus vorteilhaft sein.

Die Umsetzung verläuft gewöhnlich oberhalb von 0°C mit ausreichender Geschwindigkeit. Da sie meist unter Wärmeentwicklung verläuft, kann es von Vorteil sein, eine Kühlmöglichkeit vorzusehen.

In einigen Fällen ist es sinnvoll und vorteilhaft die Verbindungen der Formel V, insbesondere dann, wenn $R^3$ in der allgemeinen Formel V die Bedeutung einer Cyanogruppe hat, in situ zu verestern.

Die erfindungsgemäßen Ester können außerdem noch nach praktisch allen bekannten Verfahren der Estersynthese hergestellt werden, beispielsweise durch Umsetzung von entsprechenden Säureanhydriden mit den Alkoholen der Formel V, durch Umsetzung von entsprechenden Salzen mit Derivaten der Alkohole der Formel V oder durch Umesterung (vgl. Houben-Weyl a.a.0. S. 508-628).

Die als Ausgangsprodukte benötigten Alkohole der allgemeinen Formel V können für den Fall, daß $R^1$ Methyl oder Ethyl bedeutet, erhalten werden, indem man entsprechend substituierte Benzaldehyde umsetzt mit:

i) einem Reduktionsmittel im Fall von $R^3$=H,

ii) Blausäure oder einem Metallcyanid ggf. in Gegenwart einer Säure im Fall von $R^3$=CN

iii) Metallorganylen $MeR^3$ oder $R^3MeHal$ im Fall von $R^3$ = $C_2$-$C_4$-Alkinyl, $C_2$-$C_4$-Alkenyl oder $C_1$-$C_4$-Alkyl, wobei Me für ein Alkali-, Erdalkali- oder Übergangsmetall und Hal für Halogen steht.

Als Reduktionsmittel kommen alle gängigen Reduktionsmittel, die Benzaldehyde in Benzylalkohole überführen, in Frage (Houben-Weyl, Methoden der org. Chemie. Band VI/1b, S. 1 - 500, Ausgabe 1984, Georg Thieme Verlag, Stuttgart). Neben der katalytischen Hydrierung, nichtmetallischen Reduktionsmitteln und Metallhydriden sind besonders komplexe Metallhydride wie z.B. Lithiumaluminiumhydrid oder Natriumborhydrid geeignet. Kathodische- und photochemische Reduktion kommen ebenfalls in Frage.

Zur Herstellung der Cyanhydrine werden die Benzaldehyde mit Blausäure, in situ aus Metallcyaniden erzeugter Blausäure oder Metallcyaniden in Gegenwart von Alkalihydrogensulfitlösung umgesetzt, wobei gegebenenfalls basische Katalysatoren wie Kaliumcarbonat oder Phasentransferkatalysatoren wie z.B. Benzyltriethylammoniumchlorid zugegeben werden.

Als Metallcyanide werden bevorzugt Alkalimetallcyanide wie z.B. Natrium- oder Kaliumcyanid verwendet.

Die Umsetzung erfolgt in an sich bekannter Weise, z.B. wie in Houben-Weyl, Methoden der org. Chemie, Band VIII, S. 274-278, Ausgabe 1952 und Band E5, S. 1413ff, Ausgabe 1985 beschrieben.

Als Metallorganyle eignen sich die entsprechenden metallorganischen Verbindungen, insbesondere Lithiumorganylverbindungen $LiR^3$ wie Methyllithium, Ethyllithium, Butyllithium oder die entsprechenden Grignardverbindungen $R^3MgHal$, worin Hal für Chlor, Brom oder Iod steht, z.B. Methylmagnesiumbromid, Ethylmagnesiumchlorid, Propylmagnesiumiodid oder Vinylmagnesiumiodid.

Die Umsetzung mit Metallorganylen kann in an sich bekannter Weise, z.B. wie in Houben-Weyl, Methoden org. Chemie, Band 13/2a, S. 285ff, 1973 beschrieben in einem inerten organischen Lösungsmittel wie Ether oder Tetrahydrofuran unter Schutzgas durchgeführt werden, so daß sich nähere Ausführungen hierzu erübrigen.

Die Herstellung der Benzaldehyde erfolgt in der Weise, daß man entsprechend substituierte Benzonitrile mit einem Reduktionsmittel zu Aldiminen umsetzt und diese in an sich bekannter Weise verseift.

Diese Reaktionssequenz ist im folgenden Reaktionsschema dargestellt:

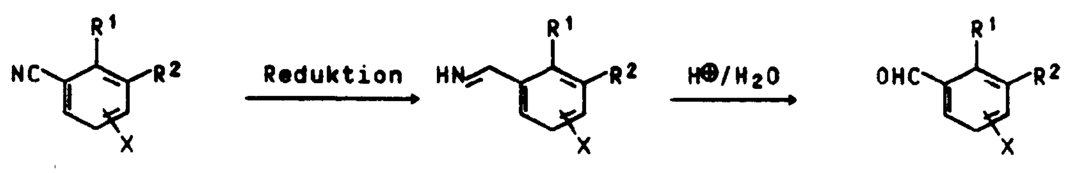


Neben Wasserstoff (katalytische Hydrierung) und Tetrachlorozinn(II)-säure sind als Reduktionsmittel besonders Aluminiumhydride wie z.B. Diisopropylaluminiumhydrid geeignet (vgl. Houben-Weyl, Methoden der organischen Chemie, Band E3, S. 476-488, Georg Thieme Verlag, Stuttgart). Die Hydrolyse der Aldimine erfolgt in der Regel durch Behandlung mit verdünnten oder konzentrierten Mineralsäuren wie Salzsäure. Bei empfindlichen Aldehyden empfiehlt sich die Verwendung von gepufferter Essigsäure.

Die Isolierung der Aldimine ist nicht unbedingt erforderlich. Die Aldimine können vorteilhaft ohne Aufarbeitung und Reinigung sofort zu den Benzaldehyden Ib hydrolysiert werden.

Zur Herstellung der Benzonitrile werden entsprechend substituiertes Chlor- oder Brombenzol mit Metallcyaniden in einem organischen Lösungsmittel gemäß folgender Reaktionsgleichung umgesetzt.

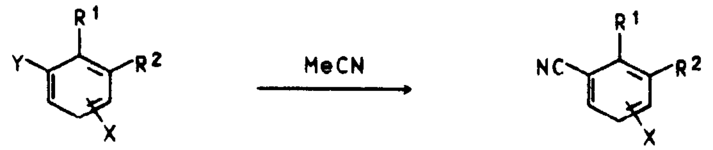


Y = Cl, Br

Als Metallcyanide (MeCN) eignen sich Alkali-, Erdalkali- und Schwermetallcyanide (Houben-Weyl, Methoden der organischen Chemie, Band E5, S. 1447-1467, Ausgabe 1985, Georg Thieme Verlag, Stuttgart). Besonders vorteilhaft ist die Verwendung von Kupfer(I)-cyanid. Die Umsetzung verläuft gut in aprotischen, polaren Lösungsmitteln. Besonders geeignet sind Dimethylformamid, Pyridin, 1-Methyl-2-pyrrolidon und Phosphorsäure-tris-(dimethylamid).

Die Reaktion wird vorteilhaft bei Temperaturen zwischen 100 und 250°C durchgeführt. Bei Verwendung von Kupfer(I)-cyanid erfolgt die Aufarbeitung des Reaktionsgemisches (Zerstörung der primär gebildeten Nitril/Kupferhalogenid/Kupfercyanid-Komplexe) entweder mit Eisen(III)-chlorid/Salzsäure, 1,2-Diamino-ethan oder Natriumcyanid. Vorteilhaft ist die Verwendung von 1,2-Diamino-ethan.

Einzelne Vertreter der Chlor- bzw. Brombenzolderivate wie 2-Chlor-6-n-butyl-toluol, 3-Chlor-2-methylstyrol, 1-Chlor-2-methyl-3-(1'-propenyl)-benzol, 2,3-Dimethylchlorbenzol, 2,3-Dimethyl-brombenzol, 4-Fluor-2,3-dimethylbrombenzol, 1,2-dichlor-3,4-dimethylbenzol, 1,5-dichlor-2,3-dimethylbenzol und 1,4-Dichlor-2,3-dimethylbenzol sind bekannt aus US-Patent 4 538 003; EP 80 359; J. Med. Chem. 28 (10), 1436-40; J. Chromatogr., 370 (3), 355-76 (1986); Gazz. Chim. Hal., 103 (8-9), 1019-25 (1973) oder Chem.-Ztg. 103 (1), 1-7 (1979).

Ein allgemeiner Syntheseweg zur Herstellung dieser Verbindungen, in der $R^1$, $R^2$, X und Y die oben angegebene Bedeutung haben, geht von Dichlor-, Dibrom-, Dibromchlor-, Dibromfluor- oder Dichlorfluorbenzolderivaten aus und wird durch folgendes Reaktionsschema verdeutlicht:

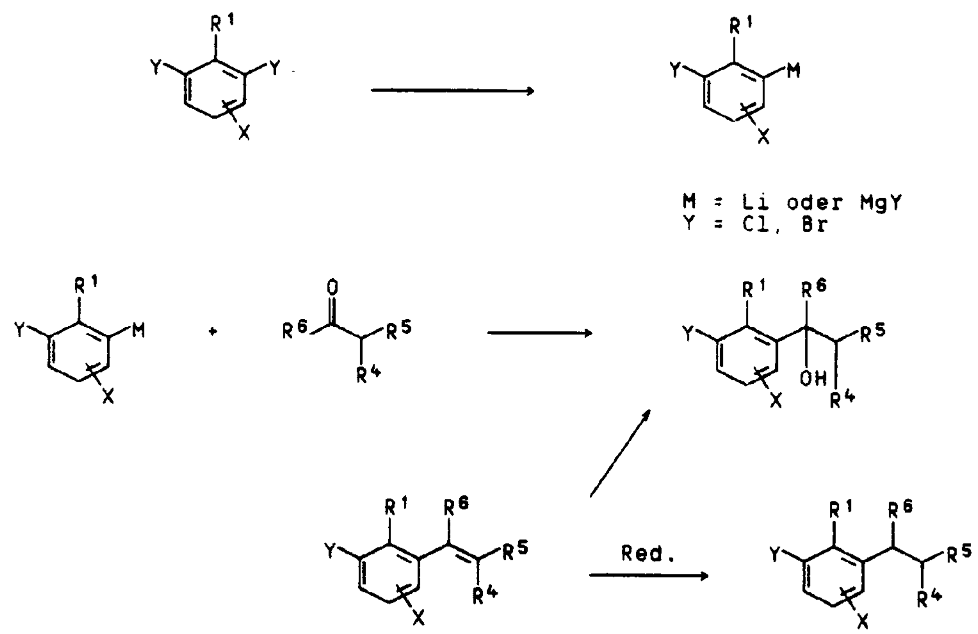


In diesem Reaktionsschema entsprechen die Seitenketten

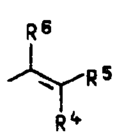 bzw. 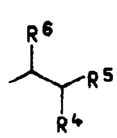 dem Rest $R^2$.

$R^4$, $R^5$ und $R^6$ haben die Bedeutung Wasserstoff, verzweigtes oder unverzweigtes Alkyl oder verzweigtes

oder unverzweigtes Alkenyl. $R^4$ und $R^6$ bzw. $R^5$ und $R^6$ können auch zu einem Ring geschlossen sein, der gegebenenfalls mit $C_1$-$C_5$-Alkylresten substituiert ist oder $R^4$, $R^5$ und $R^6$ sind derart gestaltet, daß ein bicyclisches Keton vorliegt, welches gegebenenfalls mit $C_1$-$C_5$-Alkylresten substituiert ist.

Di- bzw. Trihalogenbenzole, in denen $R^1$ Methyl oder Ethyl, X Wasserstoff, Chlor oder Fluor und Y Chlor oder Brom bedeuten, werden in die Monometallorganyle überführt. Zur Herstellung der Lithiumorgano- bzw. Grignardverbindungen kommen die gängigen Herstellverfahren, die von Arylhalogeniden ausgehen, in Frage (vgl. Houben-Weyl, Methoden der org. Chemie, Band XIII/1. S. 134 ff, Ausgabe 1970 und Band XIII/2a, S. 54 ff. Ausgabe 1973, Georg Thieme Verlag, Stuttgart). Die Synthese der Grignardverbindungen erfordert im Falle, daß Y für Chlor und X für Wasserstoff steht, höhere Reaktionstemperaturen. Besonders bewährt hat sich die Verwendung von Tetrahydrofuran bei Siedetemperatur.

Die metallorganischen Verbindungen werden mit Carbonylverbindungen VI, in denen $R^4$ bis $R^6$ die oben genannte Bedeutung haben, zu Benzylalkoholen umgesetzt. Diese werden zu Styrolen dehydratisiert, wobei die in Houben-Weyl (Methoden der org. Chemie, Band V/Ib, S. 45 ff., Ausgabe 1972, Georg-Thieme-Verlag, Stuttgart) beschriebenen Methoden in Frage kommen. Vorteilhaft ist die Verwendung saurer Dehydratisierungsmittel, insbesondere von Oxalsäure oder p-Toluolsulfonsäure, bei gleichzeitiger Entfernung des gebildeten Wasser mittels eines Abscheiders.

Die auf die vorstehend beschriebene Art und Weise erhaltenen Styrolderivate werden entweder direkt zur Herstellung der Benzonitrile eingesetzt oder zuvor reduziert. Diese Reduktion kann sowohl durch nicht katalytische Reduktion (z.B. mit Ethanol und Natrium) als auch durch katalytische Hydrierung erfolgen (vgl. Houben-Weyl, Methoden der org. Chemie, Band V/1a, S. 405 ff, Ausgabe 1970, Georg Thieme Verlag, Stuttgart). Als Katalysatoren kommen z.B. $PtO_2$, Raney-Nickel, Pd/Kohle, Pd/$CaCO_3$, Kupferchromit oder Pd/$Al_2O_3$ in Frage. Besonders bewährt hat sich die Verwendung von Pd/Kohle. Als Lösungsmittel eignen sich: Alkohole wie z.B. Methanol, Ethanol oder Isopropanol; Ether wie z.B. Tetrahydrofuran oder Dioxan; Ester wie z.B. Essigsäureethylester. Vorteilhaft ist die Verwendung von Ethanol. Die katalytische Hydrierung wird in der Regel bei Raumtemperatur und Drucken zwischen 1 und 150 bar durchgeführt. Als Nebenprodukte entstehen oft geringe Menge von Verbindungen bei denen zusätzlich ein Halogen/Wasserstoff-Austausch stattgefunden hat.

Benzylalkohole der allgemeinen Formel V, in der $R^1$ die Bedeutung Halogen hat und $R^3$ für Wasserstoff steht, können vorteilhaft durch elektrochemische Oxidation von Toluolderivaten (s. auch US-PS 3 448 021 oder J. Org. Chem. 51, 4544, 1986) in Gegenwart von Alkansäuren, z.B. Ameisen-, Essig- und Propionsäure zu Benzylestern und anschließende Hydrolyse der Benzylester gemäß folgendem Reaktionsschema hergestellt werden:

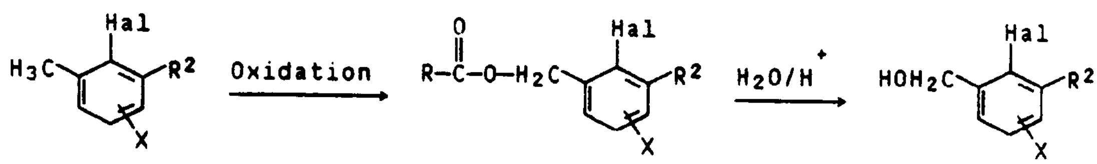


R = $C_1$-$C_3$-Alkyl

Die elektrochemische Oxydation kann in den üblichen Elektrolysezellen durchgeführt werden, bevorzugt werden ungeteilte Durchflußzellen eingesetzt. Als Anodenmaterialien dienen z.B. Edelmetalle, wie Platin oder Oxide wie $RuO_2$, $Cr_2O_3$ oder TiOx/RuOx; besonders bevorzugt wird Graphit eingesetzt. Kathodenmaterialien können u. a. Eisen, Stahl, Nickel, Edelmetalle, wie Platin oder auch Graphit sein. Als Elektrolyt dient eine Lösung der Toluole in der Alkansäure, dem zur Erhöhung der Leitfähigkeit ein Hilfselektrolyt zugesetzt wird. Weiterhin kann auch zur Erhöhung der Löslichkeit der Toluole ein Colösungsmittel zugesetzt werden. Beispiele für Colösungsmittel sind Ketone, wie Aceton, Methylethylketon, Nitrile, wie Acetonitril oder Propionitril und Anhydride, wie Acetanhydrid. Als Hilfselektrolyte können die in der Elektrochemie üblichen Leitsalze, wie z.B. Fluoride, Tetrafluoroborate, Sulfonate oder Alkylsulfate eingesetzt werden.

Der Elektrolyt hat beispielsweise folgende Zusammensetzung:

1 - 20 % Toluol der allgemeinen Formel II
1 - 10 % Leitsalz
0 - 20 % Colösungsmittel
50 - 95 % Alkansäure

Die Stromdichten und Elektrolysetemperaturen lassen sich in weiten Grenzen variieren. So wird z.B. bei 0.2 - 20 A/$dm^2$ und bei 15 bis 95°C elektrolysiert. Die eingesetzten Toluole lassen sich weitgehend umsetzen, die Aufarbeitung der Elektrolyseausträge erfolgt nach an sich bekannten Methoden, z.B. durch Destillation, Extraktion und Kristallisation, Kolösungsmittel, überschüssige Alkansäure und Leitsalz können nach Abtrennung

von den Benzylestern zusammen mit ggf. unumgesetztem Toluol der zur Elektrolyse zurückgeführt werden.

Benzylalkohole der allgemeinen Formel V, in der $R^3$ Cyano, Alkinyl, Alkenyl oder Alkyl mit 1 bzw. 2 bis 4 Kohlenstoffatomen bedeutet und $R^1$ für Halogen steht, können ausgehend von Benzaldehyden der allgemeinen Formel VI

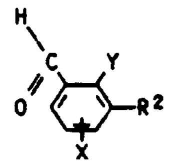

(VI),

in der Y Halogen bedeutet und $R^2$ und X die in Anspruch 1 angegebene Bedeutung haben, hergestellt werden. Dabei wird

a) wenn $R^3$ Cyano bedeutet, der Benzaldehyd VI mit Blausäure oder einem Metallcyanid gegebenenfalls in Gegenwart einer Säure zur Reaktion gebracht

b) wenn $R^3$ für Alkinyl, Alkenyl oder Alkyl mit bis zu 4 Kohlenstoffatomen steht, der Aldehyd VI mit einem entsprechenden Metallorganyl $R^3$-M umgesetzt.

Als Metallcyanide werden bevorzugt Alkalimetallcyanide wie z.B. Natrium- oder Kaliumcyanid verwendet, wobei gegebenenfalls ein Hilfsreagenz z.B. $NaHSO_3$ oder ein Phasentransferkatalysator zu dem Zweiphasensystem gefügt wird.

Als Metallorganyle eignen sich die entsprechenden metallorganischen Verbindungen mit Metallen der 1. Hauptgruppe, sowie die entsprechenden Grignardverbindungen.

Die Benzaldehyde der allgemeinen Formel VI können ausgehend von den entsprechenden Benzylalkoholen V nach literaturbekannten Methoden (Houben-Weyl, Methoden der Org. Chemie. Band E3, S. 265ff), z.B. durch Oxidation mit $MnO_2$ hergestellt werden.

Zur Herstellung von Benzylalkoholen der allgemeinen Formel V, in der $R^1$ Methoxy oder Ethoxy bedeutet, geht man von Phenolen der allgemeinen Formel VII

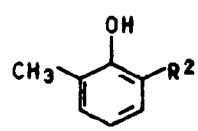

(VII)

aus. Die Phenole VII können nach den in Houben-Weyl (Methoden der org. Chemie, Band VI/3, S. 49 ff, Georg-Thieme-Verlag, Stuttgart 1965) beschriebenen Methoden alkyliert werden. Die anschließende Überführung der Methylgruppe in den Hydroxymethyl-Rest erfolgt entweder elektrochemisch oder verläuft über die entsprechenden Benzylhalogenide als Zwischenstufe. Die Benzylhalogenide können auch direkt zur Herstellung der erfindungsgemäßen Ester eingesetzt werden.

Ein anderer Syntheseweg zur Herstellung von Benzylalkoholen der allgemeinen Formel V, in der $R^1$ Methoxy oder Ethoxy bedeutet, geht von Hydroxybenzoesäuren der allgemeinen Formel VIII aus, die durch zweifache Alkylierung mit Alkylhalogeniden $R^5$-Hal

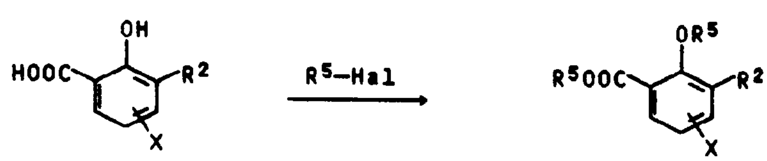


(VIII) (IX)

($R^5 = CH_3, C_2H_5$; Hal = Cl, Br, J) oder anderen gängigen Alkylierungsmitteln (vgl. Houben-Weyl, Methoden der org. Chemie, Band 6/III, S. 49 ff, Ausgabe 1965 und Band 8/III, S. 541 ff, Ausgabe 1952, Georg-Thieme-Verlag, Stuttgart) in die Alkoxybenzoesäureester IX umgewandelt werden. Die Ester IX lassen sich in an sich bekannter Weise durch Reduktion (z.B. mit komplexen Hydriden wie Lithiumaluminiumhydrid) in die gewünschten Benzylalkohole der allgemeinen Formel V, in der $R^1$ Methoxy oder Ethoxy bedeutet, überführen. Ausgehend von diesen Alkoholen können außerdem nach literaturbekannten Methoden (Houben-Weyl, Methoden der Org.

Chemie, Band E3, S. 265 ff) die entsprechenden Benzaldehyde X hergestellt werden, die sich nach den für die Benzaldehyde VI beschriebenen Methoden in α-substituierte Benzylalkohole XI überführen lassen.

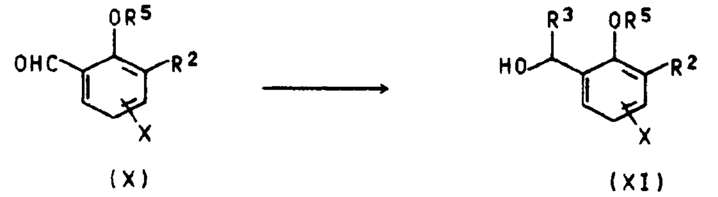

Die eingesetzten Pyrethroidsäuren und ihre Derivate sind beispielsweise in Wegler, Chemie der Pflanzenschutz- und Schädlingsbekämpfungsmittel, Band 7, Springer Verlag, Berlin, Heidelberg, New York 1981 beschrieben.

Typische Repräsentanten dieser Säuren der Formel A-H werden in der folgenden Aufzählung genannt, wobei diese Aufzählung keine Einschränkung bedeuten soll:

$A^1$-H: 3-(2′2′-Dimethylvinyl)-2,2-dimethylcyclopropan-1-carbonsäure
$A^2$-H: 3-(2′2′-Dichlorvinyl)-2,2-dimethylcyclopropan-1-carbonsäure
$A^3$-H: 3-(2′-Chlor-3′,3′,3′-trifluorprop-1′-enyl)-2,2-dimethylcyclopropan-1-carbonsäure
$A^4$-H: 3-(2′,2′-Dibromvinyl)-2,2-dimethylcyclopropan-1-carbonsäure
$A^5$-H: 3-(2′,2′-Difluorvinyl)-2,2-dimethylcyclopropan-1-carbonsäure
$A^6$-H: 3-(2′-Fluor-3′,3′,3′-trifluorprop-1′-enyl)-2,2-dimethylcyclopropan-1-carbonsäure
$A^7$-H: 3-(2′,2′-Bistrifluormethyl-vinyl)-2,2-dimethylcyclopropan-1-carbonsäure
$A^8$-H: 2-(4′-Chlorphenyl)-3-methylbuttersäure
$A^9$-H: 2-(4′-Fluorphenyl)-3-methylbuttersäure
$A^{10}$-H: 2-(4′-Difluormethoxyphenyl)-3-methylbuttersäure
$A^{11}$-H: 3-(4′-tert.-Butylphenyl)-2,2-dimethylcyclopropan-1-carbonsäure
$A^{12}$-H: 2,2,3,3,-Tetramethylcyclopropan-1-carbonsäure
$A^{13}$-H: 1-(4′-Chlorphenyl)-cyclopropan-1-carbonsäure
$A^{14}$-H: 1-(4′-Ethoxyphenyl)-2,2-dichlorcyclopropan-1-carbonsäure
$A^{15}$-H: 3-[2′-(4″-Chlorphenyl)-2′-chlorvinyl]-2,2-dimethylcyclopropan-1-carbonsäure
$A^{16}$-H: 3-(1′,3′-Butadienyl)-2,2-dimethyl-cyclopropan-1-carbonsäure
$A^{17}$-H: 3-(2′-Methyl-2′-methoxycarbonyl-vinyl)2,2-dimethylcyclopropan-1-carbonsäure
$A^{18}$-H: 2-(2′-Chlor-4′-trifluormethyl-phenylamino)-3-methylbuttersäure
$A^{19}$-H: 2-(2′-Fluor-4′-trifluormethyl-phenylamino)-3-methylbuttersäure
$A^{20}$-H: 3-Methyl-2-(4′-trifluormethyl-phenylamino)-buttersäure
$A^{21}$-H: 3-Methyl-2-(pyrrol-1′-yl)-buttersäure
$A^{22}$-H: 3-Methyl-2-(3′-methylpyrrol-1′-yl)-buttersäure
$A^{23}$-H: 2-(3′,4′-Dimethylpyrrol-1′-yl)-3-methylbuttersäure
$A^{24}$-H: 2-(2′,5′-dimethylpyrrol-1′-yl)-3-methylbuttersäure
$A^{25}$-H: 2-(Isoindolin-2-yl)-3-methylbuttersäure
$A^{26}$-H: 1,1-Dimethyl-2,2[H]indenspirocylopropan-3-carbonsäure
$A^{27}$-H: 3-Cyclopentylidenmethyl-2,2-dimethylcyclopropan-1-carbonsäure
$A^{28}$-H: 3-(1′,2′-Dibrom-2′,2′-dichlorethyl)-2,2-dimethylcyclopropan-1-carbonsäure
$A^{29}$-H: 3-Methyl-2-(pyrazol-1′-yl)-buttersäure
$A^{30}$-H: 3-Methyl-2-(imidazol-1′-yl)-buttersäure

Es versteht sich, daß die Verbindungen der Formel I in jedem Falle in Form von einheitlichen Diastereomeren, mindestens einem Paar optischer Antipoden, in vielen Fällen auch in Form von Diastereomeren-Vielfachen vorkommen und als Wirkstoffe angewandt werden können, die je nach den Ausgangsstoffen und den Umsetzungsbedingungen in einheitlicher Form oder als Gemische auftreten. Die Gemische können in üblicher Weise in ihre sterisch einheitlichen Bestandteile aufgetrennt werden; ihre biologische Wirkung ist in Einzelfällen von ihrer sterischen Konfiguration abhängig.

Die Benzylester der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Ortiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Zur Klasse der Arachnoidea gehören Spinnentiere (Acarina) beispielsweise Amblyomma americanum, Amglyomma variegatum, Argas persicus, Boophilus annulatus, Boophilus decoloratus, Boophilus microplus, Bre-

vipalpus pheonicis, Bryobia praetiosa, Dermacentor silvarum, Eotetranychus carpini, Eriophyes sheldoni, Hyalomma truncatum, Ixodes ricinus, Ixodes rubicundus Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 3 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 4 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 5. werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 10, vorzugsweise 0,05 bis 2 kg/ha.

Zu den Wirkstoffen können Öle verschiedene Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die Herstellung der erfindungsgemäßen Verbindungen erfolgt gemäß den Beschreibungen der folgenden Beispiele bzw. durch entsprechende Abwandlung dieser Beispiele.

Herstellbeispiel 1

cis, trans-3-(2′,2′-Dichlorvinyl)-2,2-dimethylcyclopropan-1-carbonsäure-(3-isopropenyl-2-methylbenzyl)ester

3,1 g 3-Isopropenyl-2-methylbenzylalkohol und 1,95 g 2-Picolin in 20 ml Toluol werden bei 20°C tropfenweise mit 4,55 g 3-(2′,2′-Dichlorvinyl)-2,2-dimethylcylcopropan-1-carbonsäurechlorid versetzt. Nach dem Abklingen der exothermen Reaktion läßt man 5 Stunden nachreagieren, gießt die Reaktionsmischung über eine mit Kieselgel gefüllte Nutsche und spült mit Toluol nach. Nach Abdestillieren des Lösungsmittels erhält man 6,4 g eines hellgelben Öls ($n^{21}$: 1,5349).

| Analyse: | | | | |
|---|---|---|---|---|
| Ber. | C 64,60 | H 6,28 | O 9,06 | Cl 20,07 |
| Gef. | C 64,80 | H 6,40 | O 9,20 | Cl 19,90 |

300-MHz-NMR-Spektrum in $CDCl_3$:

δ [ppm] = 1,15-1,35 (6H); 1,68; 1,91; 2,02 und 2,28 (2H); 2,0 (3H); 2,3 (3H); 4,83 (1H); 5,17 (2H); 5,2 (1H); 5,62 und 6,3 (1H); 7,08-7,27 (3H).

Herstellbeispiel 2

cis-3-(2′-Chlor-3′,3′,3′-trifluorprop-1′-enyl)-2,2-dimethylcyclopropan-1-carbonsäure-(3-isopropyl-2-methylbenzyl)ester und trans-3-(2′-Chlor-3′,3′,3′-trifluorprop-1′-enyl)-2,2-dimethyl-cyclopropan-1-carbonsäure-(3-isopropyl-2-methylbenzyl)ester.

4,1 g 3-Isopropyl-2-methylbenzylalkohol und 2,4 g 2-Picolin in 30 ml Toluol werden bei 20°C tropfenweise mit 6,8 g 3-(2′-Chlor-3′,3′,3′-trifluorprop-1′-enyl)-2,2-dimethylcyclopropan-1-carbonsäurechlorid [cis/trans=1:1] versetzt. Nach dem Abklingen der exothermen Reaktion läßt man 5 Stunden nachreagieren. Die Reaktionsmischung wird säulenchromatographisch über Kieselgel mit Toluol/Cyclohexan (3/7) gereinigt und gleichzeitig in den cis- und trans-Benzylester aufgetrennt (Beispiel 2a = cis-Verbindung, Beispiel 2b = trans-Verbindung).

300-MHz-NMR-Spektrum in $CDCl_3$:

cis-Verbindung (2a):

δ [ppm] = 1,22-1,37 (12H); 2,05 (1H); 2,1 (1H); 2,33 (3H); 3,25 (1H); 5,17 (2H); 6,99 (1H); 7,15-7,32 (3H).
trans-Verbindung (2b):
δ [ppm] = 1,18-1,38 (12H); 1,82 (1H); 2,32 (3H); 2,44 (1H); 3,23 (1H); 5,2 (2H); 6,14 (1H); 7,15-7,32 (3H).

Analyse der cis-Verbindung:

| | | | | |
|---|---|---|---|---|
| Ber. | C 61,78 | H 6,22 | F 14,66 | Cl 9,12 |
| Gef. | C 61,8 | H 6,3 | F 14,8 | Cl 9,1 |

Herstellbeispiel 3

3-(2′,2′-Dibromvinyl)-2,2-dimethylcyclopropan-1-carbonsäure-[3-(1′-Cyclohexenyl)-2-methyl-α-cyanobenzyl]ester

4 g 3-(1′-Cyclohexenyl)-2-methylbenzaldehyd in 100 ml Ether werden mit einer Lösung von 1,56 g Kaliumcyanid und 0,5 ml Benzyl-dimethyl-dodecylammoniumchlorid (Lutensit®) in 15 ml Wasser und 6,95 g 3-(2′,2′-Dibromvinyl)-2,2-dimethylcyclopropan-1-carbonsäurechlorid versetzt. Danach wird 15 Stunden bei Raumtemperatur gerührt, die organische Phase abgetrennt, mit Wasser gewaschen und nach Trocknung über Natriumsulfat vom Lösungsmittel befreit.

Man erhält 10,4 g eine Öles, das nach säulenchromatographischer Reinigung 7,3 g reine Verbindung ergibt.

Analyse:

| | | | | | |
|---|---|---|---|---|---|
| Ber. | C 54,46 | H 4,97 | O 6,31 | N 2,76 | Br 31,5 |
| Gef. | C 55,0 | H 5,0 | O 6,5 | N 2,8 | Br 31,2 |

200-MHz-NMR-Spektrum in $CDCl_3$:
δ [ppm] = 1,21-1,4 (6H); 1,6-2,3 (2H); 1,77 (4H); 2,21 (4H); 2,34 (3H); 5,6 (1H); 6,22 und 6,76 (1H); 6,62 (1H); 7,2-7,34 (2H); 7,5-7,6 (1H).

Herstellbeispiel 4

3-Methyl-2-(pyrrol-1′-yl)-buttersäure-(2-chlor-3-isopropylbenzyl) ester

a) Zu 35 g 2-Chlor-3-isopropylbenzylalkohol in 80 ml Tetrachlorkohlenstoff werden bei Raumtemperatur vorsichtig 29,8 g Thionylchlorid getropft. Man rührt 15 Stunden bei 25°C und engt die Reaktionslösung ein. Nach Destillation ($Kp._{0,3}$: 76-78°C) erhält man 33 g 2-Chlor-3-isopropylbenzylchlorid.

b) Eine Lösung von 7 g 3-Methyl-2-(pyrrol-1′-yl)-buttersäure in 100 ml absolutem Methanol wird mit 7,6 g Natriummethylat-Lösung (30 %ig) versetzt und 1,5 Stunden unter Rückfluß erhitzt. Nach dem Abdestillieren des Lösungsmittels versetzt man den Rückstand mit 100 ml Acetonitril, gibt 8 g 2-Chlor-3-isopropylbenzylchlorid hinzu und rührt 7 Stunden bei ca. 80°C. Das Lösungsmittel wird bei vermindertem Druck abdestilliert, der Rückstand mit Ether aufgenommen und mit Wasser gewaschen.

Nach Trocknung über Natriumsulfat und Abdestillieren des Ethers erhält man 10 g Rohprodukt. Die säulenchromatographische Reinigung über Kieselgel mit Toluol als Elutionsmittel ergibt 3,8 g des reinen Benzylesters.

200-MHz-NMR-Spectrum in $CDCl_3$:
δ [ppm] = 0,74 (3H); 0,99 (3H); 1,23 (6H); 2,43 (1H); 3,45 (1H); 4,2 (1H); 5,27 (2H); 6,17 (2H); 6,82 (2H); 7,09-7,33 (3H).

Herstellbeispiel 5

cis,trans-3-(2′-Chlor-3′,3′,3′-trifluorprop-1′-enyl)-2,2-dimethylcyclopropan-1-carbonsäure-(2-fluor-3-isopropylbenzyl)ester

5,1 g 2-Fluor-3-isopropylbenzylalkohol und 4 g Triethylamin in 100 ml Toluol werden bei Raumtemperatur tropfenweise mit 7,8 g 3-(2′-Chlor-3′, 3′, 3′-trifluorprop-1′-enyl)-2,2-dimethylcyclopropan-1-carbonsäurechlorid versetzt. Nach Abklingen der exothermen Reaktion wird 4 Stunden bei 60°C nachgerührt. Nach dem Abkühlen

wird filtriert, die Toluolphase mit Wasser gewaschen, über Natriumsulfat getrocknet und das Lösungsmittel bei vermindertem Druck abdestilliert (Rohprodukt: 14,5 g). Nach säulen- chromatographischer Reinigung über Kieselgel mit Toluol als Fließmittel erhält man 10,9 g Ester.

200-MHz-NMR-Spectrum in $CDCl_3$:

δ [ppm] = 1,18-1,32 (12H); 1,8 und 2,01 (1H); 2,15 und 2,41 (1H); 3,24 (1H); 5,2 (2H); 6,13 und 6,93 (1H); 7,03-7,32 (3H).

Herstellbeispiel 6

a) 3-sec.-Butyl-2-methyl-(α-ethinyl)benzylalkohol

50 ml THF (absolut) werden unter Stickstoffatmosphäre bei 0°C mit Acetylen gesättigt. Unter weiterem Einleiten von Acetylen tropft man innerhalb von 45 Minuten 87 ml Methylmagnesiumchlorid-Lösung (1,5 molar) zu und rührt 30 Minuten bei 0°C Bei -20°C wird eine Lösung von 15,3 g 3-sec.-Butyl-2-methylbenzaldehyd in 20 ml THF (abs.) eingetropft. Man rührt 2 Stunden bei -20°C, läßt über Nacht bei Raumtemperatur stehen und gießt die Reaktionsmischung in 300 ml Eiswasser. Nach Ansäuern mit verdünnter Salzsäure wird 3 mal mit Ether ausgeschüttelt. Die vereinigten Etherextrakte werden mit Wasser gewaschen, über Natriumsulfat getrocknet und eingeengt. Man erhält 16,6 g eines Öls (70 % gewünschte Verbindung), welches nach säulenchromatographischer Reinigung über Kieselgel mit Toluol als Fließmittel 5,1 g des reinen Benzylalkohols ergibt.

300-MHz-NMR-Spektrum in $CDCl_3$:

δ [ppm] = 0,84 (3H); 1,17 (3H); 1,57 (2H); 2,36 (3H); 2,57 (1H); 2,62 (1H); 2,97 (1H); 5,62 (1H); 7,18 (2H); 7,52 (1H).

b) 2-Chlor-3-isopropyl-(α-ethinyl)benzylalkohol

30 ml THF (absolut) werden unter Stickstoffatmosphäre bei 0-10°C mit Acetylen gesättigt. Unter weiterem Einleiten von Acetylen tropft man 81,7 ml Vinylmagnesiumchlorid-Lösung (1,47 molar) zu und rührt 30 Minuten bei 0°C. Anschließend werden 18,5 g 2-Chlor-3-isopropylbenzaldehyd eingetropft. Man rührt 15 Stunden bei Raumtemperatur und gießt die Reaktionsmischung in 300 ml Eiswasser. Nach Ansäuern mit verdünnter Salzsäure wird 3 mal mit Ether ausgeschüttelt. Die vereinigten Etherextrakte werden mit Wasser gewaschen, getrocknet und eingeengt. Es ergeben sich 22 g Rohprodukt. Nach säulenchromatographischer Reinigung über Kieselgel mit Toluol/Aceton (95/5) als Fließmittel erhält man 9 g der gewünschten Verbindung.

300-MHz-NMR-Spektrum in $CDCl_3$

δ [ppm] = 1,23 (6H); 2,64 [1H + 1H(OH)]; 3,47 (1H); 5,89 (1H); 7,3 (2H); 7,62 (1H).

Herstellbeispiel 7

cis,trans-3-(2′-Chlor-3′, 3′,3′-trifluorprop-1′-enyl)-2,2-dimethylcyclopropan-1-carbonsäure-[3-sec.-butyl-2-methyl-(α-ethinyl)benzyl]ester

2,85 g 3-sec.-Butyl-2-methyl-(α-ethinyl)benzylalkohol und 1,4 g 2-Picolin in 20 ml Toluol werden bei 20°C tropfenweise mit 3,9 g 3-(2′-Chlor-3′, 3′, 3′-trifluorprop-1′-enyl)-2,2-dimethylcyclopropan-1-carbonsäurechlorid versetzt. Nach dem Abklingen der exothermen Reaktion läßt man 15 Stunden nachreagieren, gießt die Reaktionsmischung über eine mit Kieselgel gefüllte Nutsche und spült mit Toluol nach. Nach dem Abdestillieren des Lösungsmittels erhält man 5,6 g eines Öls.

| Analyse: | | | | |
|---|---|---|---|---|
| Ber. | C 64,7 | H 6,14 | Cl 8,3 | F 13,35 |
| Gef. | C 65,3 | H 6,4 | Cl 7,8 | F 13,6 |

300-MHz-NMR-Spektrum in $CDCl_3$:

δ [ppm] = 0,84 (3H); 1,15-1,4 (9H); 1,6 (2H); 1,84; 2,06; 2,18 und teilweise verdeckte Signale (2H); 2,33 (3H); 2,63 (1H); 2,97 (1H); 5,87; 6,13 und 6,93 (1H); 6,62 (1H); 7,24 (2H); 7,51 (1H).

Herstellbeispiel 8

cis,trans-(2′,2′-Dichlorvinyl)-2,2-dimethylcyclopropan-1-carbonsäure-(2-ethoxy-3-methylbenzyl)ester

a) Herstellung der Zwischenprodukte

2-Ethoxy-3-methylbenzoesäureethylester: Zu 43,6 g Natriumhydrid (80 %ig in Weißöl) in 350 ml DMF/THF (Dimethylformamid/Tetrahydrofuran, 1:1) wird bei Raumtemperatur die Lösung von 100,3 g 2-Hydroxy-3-methyl-benzoesäure in 150 ml DMF/THF (1:1) zugetropft. Man rührt 40 Minuten bei 60°C und tropft dann bei Raumtemperatur 226,5 g Ethyliodid hinzu. Nach weiterer Zugabe von 250 ml DMF/THF (1:1) wird 9 Stunden bei 60-70°C gerührt. Man gießt die Reaktionsmischung in Eiswasser und extrahiert mit Ether. Die vereinigten organischen Phasen werden mit Wasser gewaschen, getrocknet und eingeengt. Es ergeben sich 126,3 g der gewünschten Verbindung.

200-MHz-NMR-Spektrum in $CDCl_3$:

δ [ppm] = 1,33-1,47 (6H); 2,31 (3H); 3,97 (2H); 4,37 (2H); 7,06 (1H); 7,34 (1H); 7,66 (1H).

2-Ethoxy-3-methylbenzylalkohol: Zu 24,3 g Lithiumaluminiumhydrid in 500 ml absolutem THF wird bei 20-25°C die Lösung von 125,8 g 2-Ethoxy-3-methylbenzoesäureethylester getropft. Man rührt 15 Stunden bei Raumtemperatur, tropft Eiswasser hinzu, säuert mit konzentrierter Salzsäure an und extrahiert mit Ether. Die vereinigten organischen Phasen werden mit Wasser gewaschen, getrocknet und eingeengt. Man erhält 97,1 g 2-Ethoxy-3-methylbenzylalkohol.

200-MHz-NMR-Spektrum in $CDCl_3$:

δ [ppm] = 1,42 (3H); 2,29 (3H); 3,92 (2H); 4,72 (2H); 7,01 (1H); 7,12-7,23 (2H).

b) Herstellung des Benzylesters

5,8 g 2-Ethoxy-3-methylbenzylalkohol und 4,5 g Triethylamin in 80 ml Toluol werden bei Raumtemperatur tropfenweise mit 8 g 3-(2′, 2′-Dichlorvinyl)-2,2-dimethylcyclopropan-1-carbonsäurechlorid versetzt. Nach Abklingen der exothermen Reaktion wird 6 Stunden bei 60°C gerührt. Die Toluolphase wird filtriert, mehrmals mit Wasser gewaschen, getrocknet und das Lösungsmittel bei vermindertem Druck abdestilliert. Man erhält 12,4 g des gewünschten Benzylesters.

Analyse:

| | | | |
|---|---|---|---|
| Ber. | C 60,5 | H 6,2 | Cl 19,9 |
| Gef. | C 61,6 | H 6,6 | Cl 19,6 |

300-MHz-NMR-Spektrum in $CDCl_3$:

δ [ppm] = 1,16-1,46 (9H); 1,65; 1,89; 2,04 und ein teilweise verdecktes Signal (2H); 2,3 (3H); 3,89 (2H); 5,18 (2H); 5,6 und 6,3 (1H); 7,03 (1H); 7,2 (2H).

Nach den beschriebenen Verfahren wurden die in der folgenden Tabelle genannten erfindungsgemäßen Ester hergestellt, die durch die Angabe physikalischer Eigenschaften näher charakterisiert sind; die übrigen Verbindungen der Tabelle können unter Verwendung entsprechender Ausgangsstoffe leicht erhalten werden:

Tabelle

| Nr. | $A^{n*)}$ | $R^1$ | $R^2$ | $R^3$ | X | physik. Daten $^1H$-NMR: (MHz, LM) $\delta$ [ppm] Schmelzpunkt [°C], Brechungsindex |
|---|---|---|---|---|---|---|
| 9 | $A^2$ | $CH_3$ | 1-Cyclopentenyl | H | H | |
| 10 | $A^{18}$ | $CH_3$ | 1-Cyclopentenyl | H | H | |
| 11 | $A^1$ | $CH_3$ | 1-Cyclohexenyl | H | 5-F | |
| 12 | $A^4$ | $C_2H_5$ | 1-Cyclohexenyl | H | H | |
| 13 | $A^2$ | $CH_3$ | 1-Cyclohexenyl | H | H | (200, $CDCl_3$) 1,16-1,32(6H); 1,6-1,84(4H); 1,67-2,33(2H); 2,17(4H); 2,26(3H); 5,13(2H); 5.56(1H); 5,63 und 6,3(1H); 7,05-7,3(3H) |
| 14 | $A^{12}$ | $CH_3$ | 1-Cyclohexenyl | H | H | |
| 15 | $A^{15}$ | $CH_3$ | 1-Cyclopentenyl | H | H | |
| 16 | $A^{20}$ | $CH_3$ | 1-Cyclohexenyl | H | H | 81°C |
| 17 | $A^{24}$ | $CH_3$ | 1-Cycloheptenyl | H | H | |
| 18 | $A^4$ | $CH_3$ | 1-Cyclohexenyl | H | H | (200, $CDCl_3$) 1,17-1,32(6H); 1,73 (4H); 1,95 u. mehrere verdeckte Signale (2H); 2,18(4H); 2,25(3H); 5,18(2H); 5,57(1H); 6,18 u. 6,84(1H); 7,07-7,3(3H) |
| 19 | $A^2$ | $CH_3$ | 2-Norbornen-2-yl | H | H | |
| 20 | $A^8$ | $CH_3$ | 2,5-Norbornadien-2-yl | H | H | |
| 21 | $A^3$ | $CH_3$ | 2-Norbornen-2-yl | H | H | |
| 22 | $A^4$ | $CH_3$ | 2-Norbornen-2-yl | H | H | |
| 23 | $A^1$ | $CH_3$ | 2-Norbornen-2-yl | H | H | |
| 24 | $A^8$ | $CH_3$ | 2-Norbornen-2-yl | H | H | |
| 25 | $A^1$ | $CH_3$ | 1-Cyclohexenyl | H | H | (200, $CDCl_3$) 1,11-1,32(6H); 1,47 und 3 verdeckte Signale (2H); 1,72(6H+4H); 2,17(4H); 2,28(3H); 4,9 und 5,14(1H); 5,17(2H); 5,57(1H); 7,03-7,3(3H) |
| 26 | $A^{21}$ | $C_2H_5$ | 2-Norbornen-2-yl | H | H | |
| 27 | $A^{18}$ | $C_2H_5$ | 2,5-Norbornadien-2-yl | H | H | |
| 28 | $A^2$ | $CH_3$ | 2-Norbornen-2-yl | H | 5-F | |

Tabelle (Fortsetzung)

| Nr. | $A^{n*)}$ | $R^1$ | $R^2$ | $R^3$ | X | physik. Daten $^1H$-NMR: (MHz, LM) δ [ppm] Schmelzpunkt [°C], Brechungsindex |
|---|---|---|---|---|---|---|
| 29 | $A^3$ | $CH_3$ | 1-Cyclohexenyl | H | 5-Cl | |
| 30 | $A^4$ | $CH_3$ | 1-Cyclopentenyl | H | 6-Cl | |
| 31 | $A^8$ | $CH_3$ | 1-Cyclohexenyl | H | H | (200, $CDCl_3$) 0,7(3H); 1,02(3H); 1,72(4H); 2,13(4H und 3H); 2,32(1H); 3,2(1H); 5,11(2H); 5,52(1H); 7,03-7,15(3H); 7,28(4H) |
| 32 | $A^{21}$ | $CH_3$ | 2-Norbornen-2-yl | H | H | |
| 33 | $A^{18}$ | $CH_3$ | 2-Norbornen-2-yl | H | H | |
| 34 | $A^2$ | $C_2H_5$ | 1-Cyclopentenyl | H | H | |
| 35 | $A^3$ | $CH_3$ | 2,5-Norbornadien-2-yl | H | H | |
| 36 | $A^3$ | $CH_3$ | 1-Cyclohexenyl | CN | H | (300, $CDCl_3$) 1,2-1,41(6H); 1,74(4H); 1,85; 2,07; 2,48 und 1 verdecktes Signal (2H); 2,18(4H); 2,3 (3H); 5,76(1H); 5,9; 6,17 und 6,87(1H); 6,76(1H); 7,14-7,27(2H); 7,5(1H) |
| 37 | $A^2$ | $CH_3$ | 1-Cyclohexenyl | Ethinyl | H | |
| 38 | $A^4$ | $CH_3$ | 1-Cyclohexenyl | $CH=CH_2$ | H | |
| 39 | $A^9$ | $CH_3$ | 1-Cyclohexenyl | Isopropyl | H | |
| 40 | $A^{15}$ | $CH_3$ | 1-Cyclopentenyl | CN | 5-F | |
| 41 | $A^{12}$ | $CH_3$ | 1-Cyclohexenyl | $CH_3$ | H | |
| 42 | $A^2$ | $CH_3$ | 1-Cyclohexenyl | CN | H | (200, CDCl3) 1,2-1,4(6H); 1,7(4H); 1,94 und 3 teilweise verdeckte Signale 2H); 2,2(4H); 2,33(3H); 5,58(1H); 5,63 und 6,23(1H); 6,6(1H); 7,19-7,33(2H); 7,53(1H) |
| 43 | $A^1$ | $C_2H_5$ | 1-Cyclohexenyl | CN | H | |
| 44 | $A^5$ | $C_2H_5$ | 1-Cyclohexenyl | Ethinyl | H | |
| 45 | $A^5$ | $CH_3$ | 1-Cyclohexenyl | Ethyl | 5-Cl | |
| 46 | $A^2$ | $CH_3$ | 2-Norbornen-2-yl | CN | H | |
| 47 | A2 | $CH_3$ | 2-Norbornen-2-yl | Ethinyl | H | |

Tabelle (Fortsetzung)

| Nr. | $A^n$*) | $R^1$ | $R^2$ | $R^3$ | X | physik. Daten $^1H$-NMR: (MHz, LM) δ [ppm] Schmelzpunkt [°C], Brechungsindex |
|---|---|---|---|---|---|---|
| 48 | $A^1$ | $CH_3$ | 1-Cyclohexenyl | CN | H | (200, $CDCl_3$) 1,13-1,36(6H); 1,49 und 3 teilweise verdeckte Signale (2H); 1,73(6H und 4H); 2,19(4H); 2,32(3H); 4,91 und 5,32(1H); 5,57(1H); 6,58(1H); 7,15-7,31(2H); 7,53(1H) |
| 49 | $A^2$ | $CH_3$ | 2-Norbornen-2-yl | $CH=CH_2$ | H | |
| 50 | $A^2$ | $CH_3$ | 2-Norbornen-2-yl | $CH_3$ | H | |
| 51 | $A^3$ | $CH_3$ | 2-Norbornen-2-yl | CN | H | |
| 52 | $A^4$ | $CH_3$ | 2-Norbornen-2-yl | CN | H | |
| 53 | $A^8$ | $CH_3$ | 2-Norbornen-2-yl | CN | H | |
| 54 | $A^8$ | $CH_3$ | 1-Cyclohexenyl | CN | 5-Cl | |
| 55 | $A^2$ | $CH_3$ | 2,5-Norbornadien-2-yl | CN | H | |
| 56 | $A^2$ | $CH_3$ | 2,5-Norbornadien-2-yl | Ethinyl | H | |
| 57 | $A^3$ | $CH_3$ | 2,5-Norbornadien-2-yl | CN | H | |
| 58 | $A^3$ | $CH_3$ | 2,5-Norbornadien-2-yl | Ethinyl | H | |
| 59 | $A^2$ | $CH_3$ | 1,3-Cyclohexdienyl | H | H | |
| 60 | $A^3$ trans | $CH_3$ | Cyclohexyl | H | H | (200, $CDCl_3$) 1,22-1,6(6H + 5H); 1,75-1,99(5H), 2,36(3H); 2,49 + verdecktes Signal (1H); 2,84(1H); 5,24 (2H); 5,92 und 6,19(1H); 7,2-7,38(3H) |
| 61 | $A^3$ | $CH_3$ | 1,3-Cyclohexadien-yl | H | H | |
| 62 | $A^1$ | $CH_3$ | Cyclohexyl | H | H | |
| 63 | $A^{15}$ | $CH_3$ | Cyclohexyl | H | H | |
| 64 | $A^{21}$ | $CH_3$ | Cyclohexyl | H | 5-F | |
| 65 | $A^3$ cis | $CH_3$ | Cyclohexyl | H | H | (300, $CDCl_3$) 1,22-1,53(6H + 5H); 1,74-1,92(5H); 2,02(1H); 2,16(1H); 2,3(3H); 3,78(1H); 5,15(2H); 6,65 + 6,97(1H); 7,12-7,27(3H) |
| 66 | $A^{18}$ | $CH_3$ | Cyclohexyl | H | H | |

Tabelle (Fortsetzung)

| Nr. | $A^{n*)}$ | $R^1$ | $R^2$ | $R^3$ | X | physik. Daten <br> $^1H$-NMR: (MHz, LM) δ [ppm] <br> Schmelzpunkt [°C], Brechungsindex |
|---|---|---|---|---|---|---|
| 67 | $A^2$ | $C_2H_5$ | Cyclohexyl | H | H | |
| 68 | $A^2$ | $CH_3$ | Cyclopentyl | H | H | |
| 69 | $A^3$ | $CH_3$ | Cyclopentyl | H | H | |
| 70 | $A^3$ | $CH_3$ | Cycloheptyl | H | H | |
| 71 | $A^2$ | $CH_3$ | Cyclohexyl | H | H | (200, $CDCl_3$) 1,14-1,5(6H und 5H); 1,67; 2,02 und 2 teilweise verdeckte Signale(2H); 1,84(5H); 2,31(3H); 2,8(1H); 5,18(2H); 5,63 und 6,33(1H); 7,2-7,33(3H) |
| 72 | $A^2$ | $CH_3$ | Norborn-2-yl | H | H | 1,07-2,08(15H); 2,27(1+2H); 2,25(3H); 3,46(1H); 5,12(2H); 5,61 und 6,3(1H); 7,18(2H); 7,27(1H) |
| 73 | $A^3$ | $CH_3$ | Norborn-2-yl | H | H | 1,18-2,22(15H); 2,25-2,48(1H+2H+3H); 3,45(1H); 5,17(2H); 5,87, 6,13, 6,65 und 6,96(1H); 7,15-7,32(3H) |
| 74 | $A^4$ | $CH_3$ | Norborn-2-yl | H | H | |
| 75 | $A^8$ | $CH_3$ | Norborn-2-yl | H | H | (300, $CDCl_3$); 0,69(3H); 1,01(3H); 1,17-1,66(7H); 1,88(1H); 2,14(3H); 2,25-2,4(3H); 3,18(1H); 3,42(1H); 5,0-5,2(2H); 7,06-7,33(7H) |
| 76 | $A^{18}$ | $CH_3$ | Norborn-2-yl | H | H | (200, $CDCl_3$); 0,95-1,1(6H); 1,15-1,7(7H); 1,92(1H); 2,28-2,41(3H+3H); 3,45(1H); 3,99(1H); 5,14(1H); 5,22(2H); 6,63(1H); 7,15-7,39(4H); 7,55(1H) |
| 77 | $A^4$ | $CH_3$ | Cyclohexyl | H | H | (300, $CDCl_3$) 1,18-1,54(6H u. 5H); 1,7; 2,22 und 2 verdeckte Signale (2H); 1,73-1,97(5H); 2,31(3H); 2,8(1H); 5,16 (2H); 6,16 und 6,82(1H); 7,15-7,28(3H) |
| 78 | $A^{21}$ | $CH_3$ | Norborn-2-yl | H | H | $n_D^{22}$: 1,5427 |
| 79 | $A^2$ | $CH_3$ | Cyclopropyl | H | H | (200, $CDCl_3$); 0,65(2H); 0,95(2H); 1,18-1,34(6H); 1,92(1H); 1,68; 1,92; 2,06 und 2,32(2H); 2,44(3H); 5,2(2H); 5,63 und 6,32(1H); 7,05-7,28(3H) |

Tabelle (Fortsetzung)

| Nr. | $A^{n*)}$ | $R^1$ | $R^2$ | $R^3$ | X | physik. Daten $^1H$-NMR: (MHz, LM) δ [ppm] Schmelzpunkt [°C], Brechungsindex |
|---|---|---|---|---|---|---|
| 80 | $A^1$ | $CH_3$ | Cyclopropyl | H | H | |
| 81 | $A^3$ | $CH_3$ | Cyclopropyl | H | H | (200, $CDCl_3$); 0,67(2H); 0,96(2H); 1,22-1,4(6H); 1,94(1H); 1,86; 2,08; 2,22 und 2,38(2H); 2,47(3H); 5,23(2H); 6,19 und 7,02(1H; 7,06-7,3(3H) |
| 82 | $A^4$ | $CH_3$ | Cyclopropyl | H | H | |
| 83 | $A^8$ | $CH_3$ | Cyclohexyl | H | H | (200,$CDCl_3$) 0,68(3H); 1,1(3H); 1,4(5H); 1,8(5H); 2,14(3H); 2,32(1H); 2,74(1H); 3,18(1H); 5,12(2H); 7,1-7,34(7H) |
| 84 | $A^8$ | $CH_3$ | Cyclopropyl | H | H | (200, $CDCl_3$); 0,6(2H); 0,71(3H); 0,93(2H); 1,04(3H); 1,88(1H); 2,22-2,43(1H); 2,31(3H); 3,21(1H); 5,15(2H); 7,11(3H); 7,3(4H) |
| 85 | $A^{15}$ | $CH_3$ | Cyclopropyl | H | H | |
| 86 | $A^{18}$ | $CH_3$ | Cyclopropyl | H | H | |
| 87 | $A^{21}$ | $CH_3$ | Cyclopropyl | H | H | |
| 88 | $A^{20}$ | $CH_3$ | Cyclopropyl | H | H | |
| 89 | $A^3$ | $CH_3$ | Cyclohexyl | CN | H | (200;$CDCl_3$) 1,18-1,53(6H + 5H); 1,71-1,93(5H); 2,35(3H); 2,02; 2,22; 2,44 und 1 verdecktes Signal(2H); 2,78 (1H); 6,13 und 6,86(1H); 6,56(1H); 7,17-7,49(3H) |
| 90 | $A^2$ | $CH_3$ | Cyclopropyl | CN | H | |
| 91 | $A^4$ | $CH_3$ | Cyclopropyl | Ethinyl | H | |
| 92 | $A^3$ | $CH_3$ | Cyclohexyl | Ethinyl | H | |
| 93 | $A^8$ | $CH_3$ | Cyclohexyl | $CH_3$ | H | |
| 94 | $A^{18}$ | $CH_3$ | Cyclohexyl | $CH=CH_2$ | H | |
| 95 | $A^4$ | $CH_3$ | Cyclohexyl | CN | H | (200,$CDCl_3$) 1,13-1,6 (6H + 5H); 1,63-1,92(5H); 2,01 und 3 teilweise verdeckte Signale (2H); 2,33(3H); 2,76 (1H); 6,15 und 6,70(1H); 6,55(1H); 7,2-7,48(3H) |

Tabelle (Fortsetzung)

| Nr. | $A^{n*)}$ | $R^1$ | $R^2$ | $R^3$ | X | physik. Daten $^1H$-NMR: (MHz, LM) $\delta$ [ppm] Schmelzpunkt [°C], Brechungsindex |
|---|---|---|---|---|---|---|
| 96 | $A^1$ | $CH_3$ | Norborn-2-yl | CN | H | |
| 97 | $A^2$ | $CH_3$ | Norborn-2-yl | CN | H | |
| 98 | $A^3$ | $CH_3$ | Norborn-2-yl | Ethinyl | H | |
| 99 | $A^4$ | $CH_3$ | Norborn-2-yl | Isopropyl | H | |
| 100 | $A^{18}$ | $CH_3$ | Norborn-2-yl | CN | 5-F | |
| 101 | $A^2$ | $CH_3$ | Cyclohexyl | CN | H | (300, $CDCl_3$); 1,16-1,48 (6H + 5H); 1,67; 2,12 und 2 teilweise verdeckte Signale (2H); 1,73-1,96(5H); 2,38(3H); 2,78(1H); 5,62 und 6,2(1H); 6,58(1H); 7,14-7,47(3H); |
| 102 | $A^2$ | $CH_3$ | 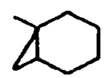 | H | H | |
| 103 | $A^4$ | $CH_3$ | 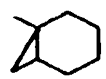 | CN | H | |
| 104 | $A^8$ | $CH_3$ | 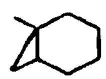 | Ethinyl | H | |
| 105 | $A^2$ | $CH_3$ | Cyclopentyl | CN | H | |
| 106 | $A^4$ | $C_2H_5$ | Cycloheptyl | CN | H | |
| 107 | $A^2$ | $CH_3$ | Isopropyl | H | H | (300, $CDCl_3$); 1,16-1,34(12H); 1,66; 1,9; 2,04 und 2,25-2,36(2H); 2,31(3H); 3,23(1H); 5,16(2H); 5,61 und 6,3(1H); 7,14-7,31(3H) |
| 108 | $A^8$ | $CH_3$ | Isopropyl | H | H | |

Tabelle (Fortsetzung)

| Nr. | $A^{n*)}$ | $R^1$ | $R^2$ | $R^3$ | X | physik. Daten $^1$H-NMR: (MHz, LM) δ [ppm] Schmelzpunkt [°C], Brechungsindex |
|---|---|---|---|---|---|---|
| 109 | $A^{15}$ | $CH_3$ | Isopropyl | H | H | (250, $CDCl_3$): 1,21(9H); 1,35(3H); 1,73(1H); 2,3(3H); 2,57(1H); 3,22(1H); 5,18(2H); 5,83(1H); 7,18(2H); 7,27(3H); 7,44(2H) |
| 110 | $A^{18}$ | $CH_3$ | Isopropyl | H | H | (200, $CDCl_3$): 0,99(6H); 1,21(6H); 2,24(3H); 3,22(1H); 4,0(1H); 5,13(1H); 5,25(2H); 6,62(1H); 7,17-7,4(4H); 7,55(1H) |
| 111 | $A^{19}$ | $CH_3$ | Isopropyl | H | H | |
| 112 | $A^{20}$ | $CH_3$ | Isopropyl | H | H | 51-56°C |
| 113 | $A^1$ | $CH_3$ | Isopropyl | H | H | (300, $CDCl_3$): 1,07-1,3(12H); 1,46; 1,88; 2,09 und 1 verdecktes Signal (2H); 1,71(6H); 2,33(3H); 3,22(1H); 4,89 und 5,4(1H); 5,12(2H); 7,11-7,28(3H) |
| 114 | $A^{21}$ | $CH_3$ | Isopropyl | H | H | $n_D^{22}$: 1,5190 |
| 115 | $A^{22}$ | $CH_3$ | Isopropyl | H | H | |
| 116 | $A^{24}$ | $CH_3$ | Isopropyl | H | H | |
| 117 | $A^2$ | $C_2H_5$ | Isopropyl | H | H | |
| 118 | $A^4$ | $CH_3$ | Isopropyl | H | 5-F | |
| 119 | $A^4$ | $CH_3$ | Isopropyl | H | H | (200, $CDCl_3$): 1,19-1,35(12H); 1,72; 1,9-2,05 und 2,25(2H); 2,33(3H); 3,26(1H); 5,2(2H); 6,2 und 6,87(1H); 7,21-7,38(3H) |
| 120 | $A^2$ | $CH_3$ | Isopropyl | H | 6-Cl | |
| 121 | $A^3$ | $CH_3$ | $-CH(C_2H_5)_2$ | H | H | (200, $CDCl_3$): 0,74(6H); 1,18-1,34(6H); 1,47-1,77(4H); 1,82; 2,03; 2,16 und 2,43(2H); 2,82(1H); 5,18(2H); 6,14 und 6,96(1H); 7,17(3H) |

Tabelle (Fortsetzung)

| Nr. | $A^{n*)}$ | $R^1$ | $R^2$ | $R^3$ | X | physik. Daten $^1H$-NMR: (MHz, LM) δ [ppm] Schmelzpunkt [°C], Brechungsindex |
|---|---|---|---|---|---|---|
| 122 | $A^2$ | $CH_3$ | $-CH(C_2H_5)_2$ | H | H | (200, $CDCl_3$); 0,78(6H); 1,15-1,3(6H); 1,44-1,76(4H); 1,9; 2,02 und zwei teilweise verdeckte Signale (2H); 2,27(3H); 2,82(1H); 5,17(2H); 5,62 und 6,3(1H); 7,17(3H) |
| 123 | $A^1$ | $CH_3$ | $-CH(C_2H_5)_2$ | H | H | |
| 124 | $A^2$ | $CH_3$ | 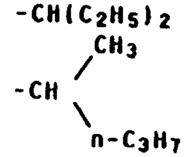 | H | H | |
| 125 | $A^2$ cis | $CH_3$ | sec-Butyl | H | H | (300,$CDCl_3$); 0,86(3H); 1,18-1,29(9H) 1,6(2H); 1,9(1H); 2,03(1H); 2,29(3H); 2,97(1H); 5,13(2H); 6,3(1H); 7,13-7,25(3H) |
| 126 | $A^3$ | $CH_3$ | 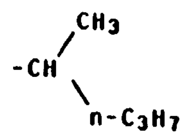 | H | H | |
| 127 | $A^{21}$ | $CH_3$ | 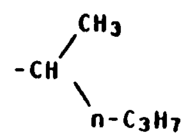 | H | H | |
| 128 | $A^8$ | $CH_3$ | $-CH(C_2H_5)_2$ | H | H | |

Tabelle (Fortsetzung)

| Nr. | $A^{n*)}$ | $R^1$ | $R^2$ | $R^3$ | X | physik. Daten<br>$^1$H-NMR: (MHz, LM) δ [ppm]<br>Schmelzpunkt [°C], Brechungsindex |
|---|---|---|---|---|---|---|
| 129 | $A^4$ | $CH_3$ | 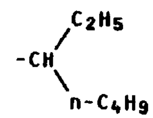 | H | H | |
| 130 | $A^2$ | $CH_3$ | 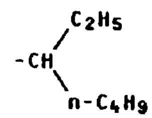 | H | H | |
| 131 | $A^2$ trans | $CH_3$ | sec-Butyl | H | H | (300, $CDCl_3$); 0,86(3H); 1,16-1,31 (9H); 1,52-1,7(2H + 1H); 2,23-2,37(1H und 3H); 2,96(1H); 5,17(2H); 5,6(1H); 7,14-7,26(3H); |
| 132 | $A^2$ | $CH_3$ | 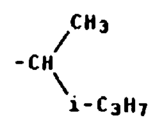 | H | H | |
| 133 | $A^4$ | $CH_3$ | 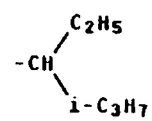 | H | H | |
| 134 | $A^8$ | $CH_3$ | $-CH(i-C_3H_7)_2$ | H | H | |
| 135 | $A^{15}$ | $CH_3$ | n-Butyl | H | H | |
| 136 | $A^3$ | $CH_3$ | n-Propyl | H | H | |
| 137 | $A^3$ cis | $CH_3$ | sec-Butyl | H | H | (300, $CDCl_3$); 0,88(3H); 1,2(3H); 1,28(6H); 1,61(2H); 2,03(1H); 2,16(1H); 2,3(3H); 2,96(1H); 5,16(2H); 6,97(1H); 7,13-7,24(3H); |

Tabelle (Fortsetzung)

| Nr. | $A^{n*)}$ | $R^1$ | $R^2$ | $R^3$ | X | physik. Daten<br>$^1$H-NMR: (MHz, LM) δ [ppm]<br>Schmelzpunkt [°C], Brechungsindex |
|---|---|---|---|---|---|---|
| 138 | $A^2$ | $CH_3$ | 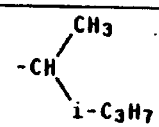 | CN | H | |
| 139 | $A^4$ | $CH_3$ | $-CH(i-C_3H_7)_2$ | Ethinyl | H | |
| 140 | $A^8$ | $CH_3$ | $-CH(C_2H_5)_2$ | $-CH=CH_2$ | H | |
| 141 | $A^{18}$ | $CH_3$ | 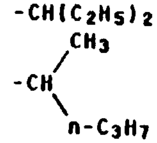 | $-CH_3$ | H | |
| 142 | $A^2$ | $CH_3$ | n-Propyl | H | H | |
| 143 | $A^3$ trans | $CH_3$ | sec-Butyl | H | H | (300,$CDCl_3$) 0,85(3H); 1,17-1,38 (3H + 6H); 1,61(2H); 1,82(1H); 2,29(3H); 2,43(1H); 2,97(1H); 5,18(2H); 5,87 und 6,15(1H); 7,14-7,25(3H) |
| 144 | $A^2$ | $CH_3$ | 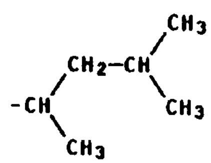 | H | H | |
| 145 | $A^4$ | $CH_3$ | 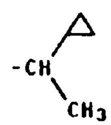 | H | H | |
| 146 | $A^{18}$ | $CH_3$ | 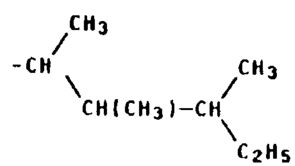 | H | H | |

Tabelle (Fortsetzung)

| Nr. | $A^{n*)}$ | $R^1$ | $R^2$ | $R^3$ | X | physik. Daten<br>$^1$H-NMR: (MHz, LM) δ [ppm]<br>Schmelzpunkt [°C], Brechungsindex |
|---|---|---|---|---|---|---|
| 147 | $A^{21}$ | $CH_3$ | 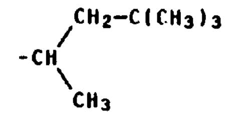 | H | H | |
| 148 | $A^{8}$ | $CH_3$ | 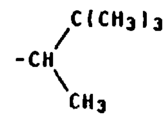 | H | H | |
| 149 | $A^{8}$ | $CH_3$ | sec-Butyl | H | H | (300, $CDCl_3$) 0,68(3H); 0,82(3H); 1,01(3H); 1,18(3H); 1,59(2H); 2,12(3H); 2,25-2,38(1H); 2,92(1H), 3,17(1H); 5,02-5,18(2H); 7,06-7,28(3H + 4H) |
| 150 | $A^{2}$ | $CH_3$ | Cyclopropyl | Ethinyl | H | |
| 151 | $A^{4}$ | $CH_3$ | Cyclopropyl | CN | H | |
| 152 | $A^{3}$ | $CH_3$ | Cyclopropyl | Isopropyl | H | |
| 153 | $A^{12}$ | $CH_3$ | 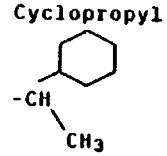 | H | H | |
| 154 | $A^{21}$ | $CH_3$ | sec-Butyl | CN | H | |
| 155 | $A^{21}$ | $CH_3$ | sec-Butyl | H | H | (300, $CDCl_3$) 0,66-1,0(9H); 1,18 (3H); 1,57(2H); 2,18(3H); 2,33-2,5(1H); 2,94(1H); 4,13(1H); 5,1-5,25(2H); 6,15 (2H); 6,75(2H); 7,07-7,23(3H) |
| 156 | $A^{2}$ | $CH_3$ | sec-Butyl | CN | H | |
| 157 | $A^{21}$ | $CH_3$ | Isopropyl | Isopropyl | H | |
| 158 | $A^{18}$ | $CH_3$ | Isopropyl | $CH=CH_2$ | 5-F | |
| 159 | $A^{4}$ | $CH_3$ | Isopropyl | Ethyl | H | |
| 160 | $A^{2}$ | $CH_3$ | Isopropyl | CN | H | |

Tabelle (Fortsetzung)

| Nr. | $A^{n*)}$ | $R^1$ | $R^2$ | $R^3$ | X | physik. Daten $^1H$-NMR: (MHz, LM) δ [ppm] Schmelzpunkt [°C], Brechungsindex |
|---|---|---|---|---|---|---|
| 161 | $A^3$ | $CH_3$ | Isopropyl | CN | H | ($300, CDCl_3$) 1,18-1,42(6H + 6H); 1,75-1,9; 2,07 und teilweise verdeckte Signale (2H); 2,37(3H);3,22(1H); 6,1-6,22 und 6,81-6,92(1H); 7,22-7,47(3H) |
| 162 | $A^2$ | $CH_3$ | Isopropyl | Ethinyl | H | |
| 163 | $A^{10}$ | $CH_3$ | 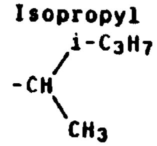 | $CH_3$ | 5-Cl | |
| 164 | $A^{15}$ | $CH_3$ | n-Butyl | $CH=CH_2$ | H | |
| 165 | $A^2$ | $CH_3$ | sec-Butyl | CN | H | |
| 166 | $A^3$ | $CH_3$ | sec-Butyl | CN | H | |
| 167 | $A^2$ | $CH_3$ | sec-Butyl | Ethinyl | H | ($300, CDCl_3$) 0,87(3H); 1,15-1,37 (9H); 1,5-1,7(2H); 1,91; 2,03 und 2 teilweise verdeckte Signale (2H); 2,3(3H); 2,64(1H); 2,98(1H); 5,6 und 6,22-6,31(1H); 6,58-6,66(1H), 7,22(2H); 7,52(1H) |
| 168 | $A^4$ | $CH_3$ | sec-Butyl | $CH=CH_2$ | H | |
| 169 | $A^{21}$ | $CH_3$ | Isopropenyl | Ethyl | H | |
| 170 | $A^{18}$ | $CH_3$ | Isopropenyl | CN | H | |
| 171 | $A^8$ | $CH_3$ | Isopropenyl | H | 5-F | |
| 172 | $A^4$ | $CH_3$ | Isopropenyl | H | H | |
| 173 | $A^3$ cis | $CH_3$ | Isopropenyl | H | H | ($300, CDCl_3$) 1,29(6H); 2,0-2,08(3H und 1H); 2,19(1H); 2,30(3H); 4,84(1H); 5,16 (2H); 5,23(1H); 6,97(1H); 7,1-7,27(3H); |
| 174 | $A^2$ | $CH_3$ | Isopropenyl | CN | H | |
| 175 | $A^2$ | $CH_3$ | Isopropenyl | Ethinyl | H | |

Tabelle (Fortsetzung)

| Nr. | $A^{n*)}$ | $R^1$ | $R^2$ | $R^3$ | X | physik. Daten<br>$^1H$-NMR: (MHz, LM) δ [ppm]<br>Schmelzpunkt [°C], Brechungsindex |
|---|---|---|---|---|---|---|
| 176 | $A^2$ | $CH_3$ | $-\overset{\overset{CH_3}{\mid}}{C}=CH-CH_3$ | H | H | |
| 177 | $A^3$ | $CH_3$ | $-\overset{\overset{C_2H_5}{\mid}}{C}=CH_2$ | H | H | |
| 178 | $A^2$ | $C_2H_5$ | Isopropenyl | H | H | |
| 179 | $A^3$ trans | $CH_3$ | Isopropenyl | H | H | (300, $CDCl_3$) 1,22(3H); 1,34(3H); 1,83(1H); 2,03(3H); 2,3(3H); 2,45(1H); 4,84(1H); 5,19 (2H); 5,22(1H); 6,15(1H); 7,1-7,27(3H); |
| 180 | $A^4$ | $CH_3$ | $-\overset{\overset{C_2H_5}{\mid}}{C}=CH-CH_3$ | H | H | |
| 181 | $A^2$ | $CH_3$ | $-\overset{\overset{C_2H_5}{\mid}}{C}=CH_2$ | H | H | |
| 182 | $A^2$ | $CH_3$ | $-\overset{\overset{C_2H_5}{\mid}}{C}=CH-CH_3$ | H | H | (300, $CDCl_3$); 0,88 und 0,98(3H); 1,15-1,30(6H); 1,33 und 1,78(3H); 1,66; 1,91; 2,04 und 1 verdecktes Signal (2H); 2,14-2,41(3H+2H); 5,13(2H); 5,29 und 5,6(1H); 5,62 und 6,3(1H); 6,97-7,26(3H) |
| 183 | $A^8$ | $CH_3$ | $-\overset{\overset{i-C_3H_7}{\mid}}{C}=CH_2$ | H | H | |
| 184 | $A^2$ | $CH_3$ | $-\overset{\overset{n-C_3H_7}{\mid}}{C}=CH-C_2H_5$ | H | H | |

Tabelle (Fortsetzung)

| Nr. | $A^{n*)}$ | $R^1$ | $R^2$ | $R^3$ | X | physik. Daten<br>$^1H$-NMR: (MHz, LM) $\delta$ [ppm]<br>Schmelzpunkt [°C], Brechungsindex |
|---|---|---|---|---|---|---|
| 185 | $A^{15}$ | $CH_3$ | $n\text{-}C_3H_7$<br>\|<br>$-C=CH_2$ | H | H | |
| 186 | $A^2$ | $CH_3$ | $CH_3$<br>\|<br>$-C=CH-CH_3$ | Ethinyl | H | |
| 187 | $A^8$ | $CH_3$ | 1-Cyclohexenyl | CN | H | (200, $CDCl_3$) 0,68-1,18(6H); 1,72 (4H); 2,02-2,22(3H u. 4H); 2,37 (1H); 3,24(1H); 5,5(1H); 6,46(1H); 7,13-7,49(7H); |
| 188 | $A^{20}$ | $CH_3$ | $CH_2-CH_2-CH=CH_2$<br>\|<br>$-C=CH_2$ | H | H | |
| 189 | $A^2$ | $CH_3$ | Vinyl | H | H | (300, $CDCl_3$); 1,15-1,3(6H); 1,63; 1,88; 2,03 und ein teilweise verdecktes Signal (2H); 2,32(3H); 5,13(2H); 5,32(1H); 5,6(1H); 5,6 und 6,28(1H); 6,94-7,05(1H); 7,13-7,3(2H); 7,45(1H) |
| 190 | $A^3$ | $CH_3$ | $C_2H_5$<br>\|<br>$-C=CH-CH_3$ | CN | H | |
| 191 | $A^2$ | $CH_3$ | $n\text{-}C_3H_7$<br>\|<br>$-C=CH-C_2H_5$ | H | H | |
| 192 | $A^{21}$ | $CH_3$ | $CH_3$<br>\|<br>$-C=CH-(i\text{-}C_3H_7)$ | H | H | |
| 193 | $A^3$ | $CH_3$ | 1-Cyclohexenyl | H | H | (200, $CDCl_3$) 1,2-1,36(6H); 1,76 (4H); 1,85; 2,05; verdecktes Signal und 2,46(2H); 2,18(4H); 2,27(3H); 5,18 (2H); 5,57(1H); 5,91; 6,16 und 6,97 (1H); 7,07-7,3(3H); |

Tabelle (Fortsetzung)

| Nr. | $A^{n*)}$ | $R^1$ | $R^2$ | $R^3$ | X | physik. Daten<br>$^1H$-NMR: (MHz, LM) $\delta$ [ppm]<br>Schmelzpunkt [°C], Brechungsindex |
|---|---|---|---|---|---|---|
| 194 | $A^8$ | $CH_3$ | $CH_2-CH(CH_3)_2$<br>\|<br>$-C=CH_2$ | H | H | |
| 195 | $A^{24}$ | $CH_3$ | 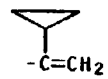<br>$-C=CH_2$ | Ethinyl | H | |
| 196 | $A^3$ | $CH_3$ | $CH_3$<br>\|<br>$-C=CH-CH_3$ | H | H | |
| 197 | $A^4$ | $CH_3$ | $C_2H_5$<br>\|<br>$-C=CH-CH_3$ | CN | H | |
| 198 | $A^3$ | $CH_3$ | Vinyl | H | H | $n_D^{22}$: 1,5065 |
| 199 | $A^2$ | $CH_3$ | $-CH=CH-CH_3$ | H | H | |
| 200 | $A^3$ | $CH_3$ | $-CH=CH-CH(CH_3)_2$ | H | H | |
| 201 | $A^4$ | $CH_3$ | $-CH=C(CH_3)_2$ | H | H | |
| 202 | $A^{18}$ | $CH_3$ | $CH_3$<br>\|<br>$-C=C(CH_3)_2$ | H | H | |
| 203 | $A^{21}$ | Cl | Isopropyl | H | H | (300, $CDCl_3$); 0,75(3H); 0,99(3H); 1,23(6H); 2,45(1H); 3,46(1H); 4,2(1H); 5,27(2H); 6,17(2H); 6,8(2H); 7,07-7,32(3H) |
| 204 | $A^2$ | Cl | Isopropyl | H | H | (300, $CDCl_3$) 1,15-1,36(12H); 1,7; 1,93; 2,05 und 2,29(2H); 3,46(1H); 5,23 (2H); 5,62 und 6,3(1H); 7,2-7,31(3H) |
| 205 | $A^4$ | Cl | Isopropyl | H | H | |
| 206 | $A^{10}$ | Cl | Isopropyl | H | H | |

Tabelle (Fortsetzung)

| Nr. | $A^{n*)}$ | $R^1$ | $R^2$ | $R^3$ | X | physik. Daten $^1H$-NMR: (MHz, LM) δ [ppm] Schmelzpunkt [°C], Brechungsindex |
|---|---|---|---|---|---|---|
| 207 | $A^{15}$ | Cl | Isopropyl | H | H | (250, $CDCl_3$): 1,22(9H); 1,37(3H); 1,78(1H); 2,57(1H); 3,45(1H); 5,3(2H); 5,85(1H); 7,26(2H+3H); 7,44(2H) |
| 208 | $A^{18}$ | Cl | Isopropyl | H | H | (300, $CDCl_3$): 1,0-1,08(6H); 1,25(6H); 2,28(1H); 3,47(1H); 4,0(1H); 5,11(1H); 5,29(2H); 6,63(1H); 7,17-7,33(4H); 7,52(1H) |
| 209 | $A^{20}$ | Cl | Isopropyl | H | H | |
| 210 | $A^3$ | Cl | Isopropyl | H | H | (300,$CDCl_3$) 1,2-1,36(12H); 1,87; 2,08; 2,2 und 2,47(2H); 3,47(1H); 5,26 (2H); 6,17 und 6,97(1H); 7,23-7,32(3H) |
| 211 | $A^{24}$ | Cl | Isopropyl | H | H | |
| 212 | $A^{19}$ | Cl | Isopropyl | H | H | |
| 213 | $A^{23}$ | Cl | Isopropyl | H | H | |
| 214 | $A^2$ | Cl | tert.-Butyl | H | H | (300, $CDCl_3$): 1,19-1,32(6H); 1,52(9H); 1,71; 1,95; 2,07 und 2,3(2H); 5,27(2H); 5,62 und 6,3(1H); 7,18-7,3(2H); 7,42(1H) |
| 215 | $A^3$ | Cl | tert-Butyl | H | H | |
| 216 | $A^1$ | Cl | Isopropyl | H | H | (300,$CDCl_3$) 1,11-1,32(6H); 1,5; 1,93; 2,11 und 1 teilweise verdecktes Signal (2H); 1,72(6H); 3,47(1H); 4,92 und 5,4(1H); 5,25(2H); 7,26(3H) |
| 217 | $A^2$ | Cl | Isopropyl | H | 5-Cl | |
| 218 | $A^3$ | Cl | Isopropyl | H | 6-Cl | |
| 219 | $A^{15}$ | Cl | tert-Butyl | H | H | |
| 220 | $A^{21}$ | Cl | tert.-Butyl | H | H | (200, $CDCl_3$); 0,76(3H); 0,99(3H); 1,5(9H); 2,46(1H); 4,21(1H); 5,3(2H); 6,18(2H); 6,82(2H); 7,17(2H); 7,43(1H) |

Tabelle (Fortsetzung)

| Nr. | $A^{n*)}$ | $R^1$ | $R^2$ | $R^3$ | X | physik. Daten<br>$^1$H-NMR: (MHz, LM) $\delta$ [ppm]<br>Schmelzpunkt [°C], Brechungsindex |
|---|---|---|---|---|---|---|
| 221 | $A^{20}$ | Cl | sec-Butyl | H | H | |
| 222 | $A^2$ | Cl | $-CH(C_2H_5)_2$ | H | H | |
| 223 | $A^8$ | Cl | Isopropyl | H | H | (300, $CDCl_3$) 0,7(3H); 1,03(3H); 1,22(6H); 2,33(1H); 3,22(1H); 3,44(1H); 5,12-5,3(2H); 7,03-7,32(3H + 4H) |
| 224 | $A^2$ | Cl | Isopropyl | H | 6-Cl | |
| 225 | $A^3$ | Cl | Ethyl | H | H | (300, $CDCl_3$); 1,2-1,38(6H+3H); 1,78; 1,86; 1,98; 2,07; 2,19 und 2,46(2H); 2,8(2H); 5,25(2H); 5,88; 6,16; 6,64 und 6,95(1H); 7,24(3H) |
| 226 | $A^2$ | Cl | Ethyl | H | H | |
| 227 | $A^{21}$ | Cl | Ethyl | H | H | |
| 228 | $A^{24}$ | Cl | Ethyl | H | H | |
| 229 | $A^2$ | Cl | Cyclopentyl | H | H | |
| 230 | $A^3$ | Cl | Cyclopentyl | H | H | (300, $CDCl_3$); 1,2-1,38(6H); 1,45-2,5(8H+2H); 3,49(1H); 5,26(2H); 5,89; 6,17; 6,64 und 6,95(1H); 7,12-7,33(3H) |
| 231 | $A^2$ | Cl | Isopropyl | Ethinyl | H | (200; $CDCl_3$) 1,15-1,34(12H); 1,68; 1,93; 2,05 und 2,22-2,37(2H); 2,68(1H); 3,49(1H); 5,63 und 6,3(1H); 6,82(1H); 7,38(2H); 7,7(1H); |
| 232 | $A^2$ | Cl | sec.-Butyl | H | H | (200, $CDCl_3$); 0,82(3H); 1,15-1,3(9H); 1,5-2,32(4H); 3,27(1H); 5,24(2H); 5,61 und 6,29(1H); 7,25(3H) |
| 233 | $A^3$ | Cl | sec.-Butyl | H | H | (200, $CDCl_3$); 0,82(3H); 1,17-1,38(9H); 1,6(2H); 1,96; 2,07; 2,19 und 2,45(2H); 3,28(1H); 5,25(2H); 6,16 und 6,94(1H); 7,23(3H) |
| 234 | $A^2$ | Cl | Cyclopentyl | CN | H | |
| 235 | $A^3$ | Cl | Ethyl | Ethinyl | H | |

Tabelle (Fortsetzung)

| Nr. | $A^{n*)}$ | $R^1$ | $R^2$ | $R^3$ | X | physik. Daten<br>$^1H$-NMR: (MHz, LM) δ [ppm]<br>Schmelzpunkt [°C], Brechungsindex |
|---|---|---|---|---|---|---|
| 236 | $A^{21}$ | Cl | Cyclopentyl | H | H | (300, $CDCl_3$); 0,73(3H); 0,98(3H); 1,55(2H); 1,75(4H); 2,08(2H); 2,45(1H); 3,48(1H); 4,2(1H); 5,25(2H); 6,15(2H); 6,78(2H); 7,04-7,3(3H) |
| 237 | $A^2$ | Cl | Cycloheptyl | H | H | |
| 238 | $A^3$ | Cl | Cyclohexyl | H | H | (200, $CDCl_3$); 1,31-1,62(6H+6H); 1,83-2,63(4H+2H); 3,2(1H); 5,38(2H); 6,01; 6,28; 6,77 und 7,07(1H); 7,38(3H) |
| 239 | $A^3$ | Cl | Isopropyl | Ethinyl | H | (300,$CDCl_3$) 1,16-1,38(12H); 1,75-1,85; 2,02; 2,2 und 2,47(2H); 2,66(1H);3,46(1H); 5,84; 6,14 und 6,93(1H); 7,33(2H); 7,65(1H) |
| 240 | $A^{19}$ | Cl | Cyclohexyl | Ethinyl | H | |
| 241 | $A^2$ | Cl | Cyclohexyl | H | H | (200, $CDCl_3$); 1,21-2,41(10H+6H+2H); 3,1(1H); 5,28(2H); 5,65 und 6,31(1H); 7,3(3H) |
| 242 | $A^{21}$ | Cl | Cyclohexyl | H | H | |
| 243 | $A^2$ | Cl | Cyclohexyl | CN | H | |
| 244 | $A^{21}$ | Cl | Isopropyl | Ethinyl | H | |
| 245 | $A^2$ | Cl | Cyclopropyl | H | H | |
| 246 | $A^3$ | Cl | Isopropyl | CN | H | |
| 247 | $A^2$ | Cl | Isopropyl | CN | H | (200,$CDCl_3$) 1,2-1,38(6H und 6H); 1,72; 1,96; 2,16 und 2,33(2H); 3,5(1H); 5,66 und 6,17-6,31(1H); 6,8(1H); 7,34-7,51(2H); 7,62(1H) |
| 248 | $A^3$ | Cl | Cyclopropyl | H | H | |
| 249 | $A^{21}$ | Cl | Cyclopropyl | H | H | |

Tabelle (Fortsetzung)

| Nr. | $A^{n*)}$ | $R^1$ | $R^2$ | $R^3$ | X | physik. Daten<br>$^1H$-NMR: (MHz, LM) δ [ppm]<br>Schmelzpunkt [°C], Brechungsindex |
|---|---|---|---|---|---|---|
| 250 | $A^{21}$ | F | Isopropyl | H | H | (300, $CDCl_3$); 0,74(3H); 0,95(3H); 1,25(6H); 2,42(1H); 3,23(1H); 4,18(1H); 5,2(2H); 6,15(2H); 6,79(2H); 7,06(2H); 7,23(1H) |
| 251 | $A^2$ | F | tert.-Butyl | H | H | (200, $CDCl_3$); 1,18-1,31(6H); 1,41(9H); 1,68; 1,9; 2,05 und 2,28(2H); 5,22(2H); 5,62 und 6,3(1H); 7,09(1H); 7,3(2H) |
| 252 | $A^3$ | F | Cyclopentyl | H | H | |
| 253 | $A^2$ | F | Cyclopropyl | H | H | |
| 254 | $A^{21}$ | F | Cyclohexyl | H | H | |
| 255 | $A^2$ | F | Ethyl | H | H | |
| 256 | $A^2$ | F | iso-Propyl | CN | H | (300, $CDCl_3$); 1,18-1,38(12H); 1,69; 1,91; 2,12 und 2,32(2H); 3,28(1H); 5,62 und 6,2(1H); 6,63(1H); 7,2(1H); 7,35-7,5(2H) |
| 257 | $A^2$ | F | Isopropyl | H | H | (200,$CDCl_3$) 1,14-1,31(12H); 1,67; 1,88; 2,04 und 2,26(2H); 3,27(1H); 5,2 (2H); 5,62 und 6,3(1H); 7,05-7,32(3H) |
| 258 | $A^2$ | F | Methyl | H | H | |
| 259 | $A^2$ | Cl | Methyl | H | H | |
| 260 | $A^2$ | F | sec-Butyl | H | H | |
| 261 | $A^2$ | F | Cyclopentyl | H | H | |
| 262 | $A^3$ | F | tert-Butyl | H | H | |
| 263 | $A^3$ | F | Cyclopropyl | H | H | |
| 264 | $A^3$ | F | Cyclohexyl | H | H | |
| 265 | $A^{21}$ | F | Ethyl | H | H | |
| 266 | $A^{21}$ | F | Cyclopropyl | H | H | |

Tabelle (Fortsetzung)

| Nr. | $A^{n*)}$ | $R^1$ | $R^2$ | $R^3$ | X | physik. Daten<br>$^1H$-NMR: (MHz, LM) δ [ppm]<br>Schmelzpunkt [°C], Brechungsindex |
|---|---|---|---|---|---|---|
| 267 | $A^2$ | F | $-CH(C_2H_5)_2$ | Isopropyl | H | |
| 268 | $A^3$ trans | Cl | Isopropyl | H | H | (300, $CDCl_3$) 1,19-1,38(12H); 1,87 (1H); 2,47(1H); 3,47(1H); 5,27(2H); 6,17(1H); 7,26(3H); |
| 269 | $A^3$ | Cl | Norborn-2-yl | H | H | |
| 270 | $A^2$ | F | Norborn-2-yl | H | H | |
| 271 | $A^2$ | Cl | 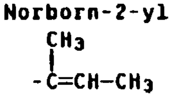 | H | H | |
| 272 | $A^{21}$ | F | 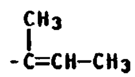 | H | H | |
| 273 | $A^2$ | Cl | 2-Cyclohexenyl | H | H | |
| 274 | $A^{15}$ | F | 1-Cyclohexenyl | H | H | |
| 275 | $A^3$ | F | Norborn-2-yl | H | H | |
| 276 | $A^2$ | Cl | Norborn-2-yl | H | H | |
| 277 | $A^{21}$ | Cl | Norborn-2-yl | H | H | |
| 278 | $A^2$ | Cl | 2-Cyclopentenyl | H | H | |
| 279 | $A^2$ | $OC_2H_5$ | Cyclohexyl | H | H | (300, $CDCl_3$) 1,14-1,53(14H); 1,65; 2,03; 2,23-2,35 und teilweise verdecktes Signal (2H); 1,7-1,93 (5H); 2,92(1H); 3,77-3,94(2H); 5,19 (2H); 5,61 und 6,31(1H); 7,03-7,28 (3H) |
| 280 | $A^3$ | Cl | 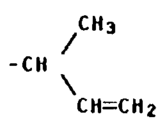 | H | H | |
| 281 | $A^2$ | F | 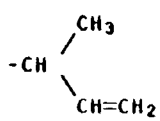 | H | H | |

Tabelle (Fortsetzung)

| Nr. | $A^{n*)}$ | $R^1$ | $R^2$ | $R^3$ | X | physik. Daten<br>$^1H$-NMR: (MHz, LM) δ [ppm]<br>Schmelzpunkt [°C], Brechungsindex |
|---|---|---|---|---|---|---|
| 282 | $A^3$ | Cl | $CH_3$<br>\|<br>$-C=CH-CH_3$ | H | H | |
| 283 | $A^2$ | F | $CH_3$<br>\|<br>$-C=CH-CH_3$ | H | H | |
| 284 | $A^{21}$ | Cl | Cyclohexyl | H | 5-Cl | |
| 285 | $A^4$ | Cl | 2-Cyclopentenyl | H | H | |
| 286 | $A^2$ | Br | Isopropyl | H | H | (300, $CDCl_3$); 1,17-1,31(12H); 1,7; 2,95; 2,07 und 2,29(2H); 3,46(1H); 5,23(2H); 5,62 und 6,3(1H); 7,27(3H); |
| 287 | $A^3$ | Br | Isopropyl | H | H | (300, $CDCl_3$); 1,2-1,37(12H); 1,8; 1,87; 2,07; 2,2 und 2,46(2H); 3,48(1H); 5,26(2H); 5,89; 6,17 und 6,95(1H); 7,28(3H); |
| 288 | $A^{21}$ | Br | Isopropyl | H | H | (300, $CDCl_3$); 0,76(3H); 0,98(3H); 1,24(6H); 2,47(1H); 3,46(1H); 4,2(1H); 5,25(2H); 6,18(2H); 6,8(2H); 7,07(1H); 7,24(2H); |
| 289 | $A^2$ | Br | Cyclopentyl | H | H | |
| 290 | $A^{15}$ | Br | tert-Butyl | H | H | |
| 291 | $A^3$ trans | $OC_2H_5$ | Methyl | H | H | (300,$CDCl_3$) 1,19-1,45(9H); 1,82(1H); 2,3(3H); 2,45(1H); 3,91 (2H); 5,22(2H); 6,13(1H); 7,02(1H); 7,14-7,26(2H) |
| 292 | $A^3$ | $OC_2H_5$ | Cyclohexyl | H | H | |
| 293 | $A^{21}$ | $OC_2H_5$ | Cyclopropyl | H | H | |
| 294 | $A^4$ | $OC_2H_5$ | Cyclohexyl | CN | H | |
| 295 | $A^{15}$ | $OC_2H_5$ | Cyclohexyl | Ethinyl | H | |
| 296 | $A^2$ | Br | Methyl | H | H | |

Tabelle (Fortsetzung)

| Nr. | $A^{n*)}$ | $R^1$ | $R^2$ | $R^3$ | X | physik. Daten $^1H$-NMR: (MHz, LM) δ [ppm] Schmelzpunkt [°C], Brechungsindex |
|---|---|---|---|---|---|---|
| 297 | $A^2$ | Br | Cyclopropyl | H | H | |
| 298 | $A^{21}$ | $OC_2H_5$ | Cyclohexyl | H | H | |
| 299 | $A^{21}$ | $OC_2H_5$ | Methyl | H | H | |
| 300 | $A^3$ trans | $OCH_3$ | Methyl | H | H | (300, $CDCl_3$) 1,22(3H); 1,34 (3H); 1,82(1H); 2,34(3H); 2,43(1H); 3,78(3H); 5,23(2H); 6,14(1H); 7,04 (1H); 7,16-7,32(2H) |
| 301 | $A^{15}$ | $OCH_3$ | Methyl | H | H | |
| 302 | $A^2$ | $OCH_3$ | Cyclohexyl | H | H | (300, $CDCl_3$); 1,15-1,32(6H); 1,42(6H); 1,63-2,38(2H+4H); 2,94(1H); 3,77(3H); 5,2(2H); 5,6 und 6,31(1H); 7,07-7,28(3H) |
| 303 | $A^3$ | $OCH_3$ | Cyclohexyl | H | H | |
| 304 | $A^2$ | Br | tert-Butyl | H | H | |
| 305 | $A^3$ | Br | sec-Butyl | H | H | |
| 306 | $A^{21}$ | Br | Cyclohexyl | H | H | |
| 307 | $A^2$ | $OCH_3$ | tert-Butyl | H | H | |
| 308 | $A^3$ | $OCH_3$ | tert-Butyl | H | H | |
| 309 | $A^2$ | $OCH_3$ | Methyl | H | H | (300, $CDCl_3$) 1,16-1,35(6H); 1,64; 1,88, 2,03 und ein teilweise verdecktes Signal (2H); 2,33(3H); 3,77 (3H); 5,19(2H); 5,6 und 6,3(1H); 7,03 (1H); 7,14-7,26(2H); |
| 310 | $A^8$ | $OCH_3$ | tert-Butyl | H | H | |
| 311 | $A^2$ | $OC_2H_5$ | tert-Butyl | H | H | |
| 312 | $A^3$ | $OC_2H_5$ | Isopropyl | H | H | |
| 313 | $A^2$ | $OC_2H_5$ | Isopropyl | H | H | |
| 314 | $A^{21}$ | $OC_2H_5$ | Isopropyl | H | H | |
| 315 | $A^3$ | $OC_2H_5$ | tert-Butyl | H | H | |

Tabelle (Fortsetzung)

| Nr. | $A^{n*)}$ | $R^1$ | $R^2$ | $R^3$ | X | physik. Daten<br>$^1H$-NMR: (MHz, LM) δ [ppm]<br>Schmelzpunkt [°C], Brechungsindex |
|---|---|---|---|---|---|---|
| 316 | $A^2$ | $OCH_3$ | Isopropyl | H | H | |
| 317 | $A^3$ | $OCH_3$ | Isopropyl | H | H | |
| 318 | $A^{21}$ | $OCH_3$ | Isopropyl | H | H | |
| 319 | $A^2$ | $OCH_3$ | Cyclopropyl | H | H | |
| 320 | $A^2$ | Cl | Isopropenyl | H | H | |
| 321 | $A^3$ | Cl | Isopropenyl | H | H | |
| 322 | $A^2$ | F | Isopropenyl | H | H | |
| 323 | $A^{21}$ | F | Isopropenyl | H | H | |
| 324 | $A^2$ | $CH_3$ | 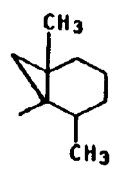 | H | H | |
| 325 | $A^4$ | $CH_3$ | 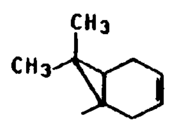 | H | H | |
| 326 | $A^2$ | Cl | 3,5-Diethylcyclohexyl | H | H | |
| 327 | $A^2$ | $CH_3$ | $-CH=C(CH_3)_2$ | H | H | (200, $CDCl_3$); 1,17-1,32(6H); 1,62(3H); 1,9(3H); 1,86-2,3(2H); 2,21(3H); 5,13(2H); 5,6 und 6,3(1H); 6,22(1H); 7,1-7,27(3H); |
| 328 | $A^{20}$ | F | Isopropyl | H | H | (200, $CDCl_3$); 1,01(6H); 1,27(6H); 2,17(1H); 3,28(1H); 3,96(1H); 5,26(2H); 6,65(2H); 7,2-7,47(5H); Fp: 79°C |

Tabelle (Fortsetzung)

| Nr. | $A^{n*)}$ | $R^1$ | $R^2$ | $R^3$ | X | physik. Daten<br>$^1$H-NMR: (MHz, LM) δ [ppm]<br>Schmelzpunkt [$^0$C], Brechungsindex |
|---|---|---|---|---|---|---|
| 329 | $A^{20}$ | Cl | Isopropyl | H | H | 57-59$^0$C |
| 330 | $A^2$ | $CH_3$ | Isopropenyl | $CH=CH_2$ | H | $n_D^{22}$: 1,5341 |
| 331 | $A^3$ | $CH_3$ | Isopropenyl | $CH=CH_2$ | H | $n_D^{22}$: 1,4958 |
| 332 | $A^{20}$ | $CH_3$ | Norborn-2-yl | H | H | (300, $CDCl_3$): 0,98(6H); 1,18-1,67(7H); 1,91(1H); 2,1-2,41(3H); 2,21(3H); 3,42(1H); 3,93(1H); 4,46(1H); 5,19(2H); 6,63(2H); 7,17(2H); 7,28(1H); 7,37(2H) |
| 333 | $A^{20}$ | $CH_3$ | Isopropenyl | H | H | 98$^0$C |
| 334 | $A^{21}$ | $CH_3$ | Isopropenyl | H | H | $n_D^{22}$: 1,5263 |
| 335 | $A^2$ | $CH_3$ | $C_2H_5$<br>$-C=CH-CH_3$ | CN | H | $n_D^{22}$: 1,5315 |
| 336 | $A^3$ | $CH_3$ | $C_2H_5$<br>$-C=CH-CH_3$ | H | H | $n_D^{22}$: 1,4955 |
| 337 | $A^1$ | $CH_3$ | $C_2H_5$<br>$-C=CH-CH_3$ | H | H | $n_D^{22}$: 1,5151 |
| 338 | $A^8$ | $CH_3$ | $C_2H_5$<br>$-C=CH-CH_3$ | H | H | $n_D^{22}$: 1,5370 |
| 339 | $A^{20}$ | $CH_3$ | $C_2H_5$<br>$-C=CH-CH_3$ | H | H | 83$^0$C |
| 340 | $A^{21}$ | $CH_3$ | $C_2H_5$<br>$-C=CH-CH_3$ | H | H | $n_D^{22}$: 1,5229 |

Tabelle (Fortsetzung)

| Nr. | $A^{n*)}$ | $R^1$ | $R^2$ | $R^3$ | X | physik. Daten<br>$^1$H-NMR: (MHz, LM) $\delta$ [ppm]<br>Schmelzpunkt [°C], Brechungsindex |
|---|---|---|---|---|---|---|
| 341 | $A^6$ | $CH_3$ | Vinyl | H | H | $n_D^{22}$: 1,5535 |
| 342 | $A^{28}$ | $CH_3$ | Isopropyl | H | H | (200, $CDCl_3$); 1,13-1,42(12H); 1,7; 1,95 und 2 teilweise verdeckte Signale (2H); 2,26(3H); 3,23(1H); 4,26-4,52(1H); 5,19(2H); 7,18-7,36(3H) |
| 343 | $A^{18}$ | F | Isopropyl | H | H | $n_D^{23}$: 1,5050 |
| 344 | $A^{18}$ | $CH_3$ | Isopropenyl | H | H | $n_D^{23}$: 1,5220 |
| 345 | $A^{29}$ | $CH_3$ | $-\overset{C_2H_5}{\overset{\vert}{C}}=CH-CH_3$ | H | H | $n_D^{23}$: 1,5196 |
| 346 | $A^{29}$ | $CH_3$ | Isopropyl | H | H | $n_D^{23}$: 1,5132 |
| 347 | $A^{18}$ | $CH_3$ | $-\overset{C_2H_5}{\overset{\vert}{C}}=CH-CH_3$ | H | H | (300, $CDCl_3$); 0,84-1,12(3H+6H); 1,3 und 1,78(3H); 2,1-2,4(3H+1H+2H); 3,98(1H); 5,08-5,32 und 5,57(2H+1H+1H); 6,6(1H); 6,98-7,34(4H); 7,49(1H) |
| 348 | $A^{18}$ | Cl | tert.-Butyl | H | H | (300, $CDCl_3$); 1,04(6H); 1,5(9H); 2,26(1H); 4,0(1H); 5,06(1H); 5,3(2H); 6,62(1H); 7,2(2H); 7,31(1H); 7,44(1H); 7,52(1H) |
| 349 | $A^{20}$ | Cl | tert.-Butyl | H | H | (300, $CDCl_3$); 1,02(6H); 1,48(9H); 1,19(1H); 3,97(1H); 4,48(1H); 5,29(2H); 6,64(2H); 7,18(2H); 7,37-7,43(3H); |
| 350 | $A^2$ | $CH_3$ | $-CH(C_2H_5)_2$ | CN | H | (200, $CDCl_3$); 0,81(6H); 1,22-1,4(6H); 1,47-1,85(4H); 1,98; 2,15 u. 2 teilweise verdeckte Signale (2H); 2,36(3H); 2,88(1H); 5,69 u. 6,27(1H); 6,64(1H); 7,33(2H); 7,5(1H); |

Tabelle (Fortsetzung)

| Nr. | $A^{n*)}$ | $R^1$ | $R^2$ | $R^3$ | X | physik. Daten<br>$^1H$-NMR: (MHz, LM) δ [ppm]<br>Schmelzpunkt [°C], Brechungsindex |
|---|---|---|---|---|---|---|
| 351 | $A^8$ | $CH_3$ | $-CH(C_2H_5)_2$ | H | H | (200, $CDCl_3$); 0,65-0,8(3H + 6H); 1,0(3H); 1,43-1,78(4H); 2,13(3H); 2,32(1H); 2,78(1H); 3,2(1H); 5,14(2H);7,13(3H); 7,29(4H); |
| 352 | $A^{18}$ | $CH_3$ | $-CH(C_2H_5)_2$ | H | H | (300, $CDCl_3$); 0,77(6H); 0,93-1,07(6H); 1,53(2H);, 1,67(2H); 2,22(3H + 1H); 2,79(1H); 3,98(1H); 5,13(1H); 5,22(2H); 6,6(1H); 7,13(3H); 7,3(1H); 7,49(1H); |
| 353 | $A^2$ | $CH_3$ | 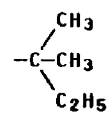 | CN | H | (200, $CDCl_3$) 0,67(3H); 1,18-1,35(6H); 1,4(6H); 1,71; 2,13; 2,32 u. ein verdecktes Signal (2H); 1,83(2H); 2,48(3H); 5,61 u. 6,2(1H); 6,6(1H); 7,17-7,27(1H); 7,4-7,55(2H); |
| 354 | $A^2$ | $CH_3$ | 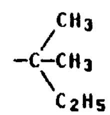 | H | H | (200, $CDCl_3$) 0,67(3H); 1,14-1,3(6H); 1,41(6H); 1,66; 2,02; 2,27 u. ein verdecktes Signal (2H); 1,83(2H); 2,43(3H); 5.14(2H); 5,6 u. 6,29(1H); 7,07-7,27(2H); 7,32(1H); |
| 355 | $A^3$ | $CH_3$ | 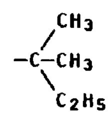 | H | H | (200, $CDCl_3$) 0,67(3H); 1,17-1,36(6H); 1,4(6H); 1,83(2H); 1,43(3H); 2,02; 2,18; 2,38-2,53 u. ein teilweise verdecktes Signal (2H); 5,17(2H); 6,12 u. 6,95 (1H); 7,08-7,23(2H); 7,32(1H) |
| 356 | $A^8$ | $CH_3$ | 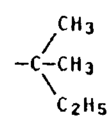 | H | H | (200, $CDCl_3$) 0,65(3H + 3H); 1,02(3H); 1,35(3H); 1,8(2H); 2,21-2,4(1H); 2,28(3H); 3,18(1H); 5,09(2H); 7,05-7,37(7H); |

Tabelle (Fortsetzung)

| Nr. | $A^{n*)}$ | $R^1$ | $R^2$ | $R^3$ | X | physik. Daten<br>$^1$H-NMR: (MHz, LM) δ [ppm]<br>Schmelzpunkt [°C], Brechungsindex |
|---|---|---|---|---|---|---|
| 357 | $A^2$ | $CH_3$ | Isopropyl | $CH_3$ | H | (300, $CDCl_3$); 1,1-1,32(12H); 1,5(3H); 1,87; 1,9; 2,0 u. ein teilweise verdecktes Signal (2H); 2,33(3H); 3,22(1H); 5,6 u. 6,3(1H); 6,18(1H); 7,11-7,32(32H); |
| 358 | $A^3$ | $CH_3$ | Isopropyl | $CH_3$ | H | (300, $CDCl_3$); 1,17-138(12H); 1,52(3H); 1,83; 2,04; 2,14 u. ein verdecktes Signal (2H); 2,31(3H); 3,23(1H); 6,2(1H); 6,2; 7,89 u. 7,98(1H); 7,12-7,34(3H); |
| 359 | $A^8$ | $CH_3$ | Isopropyl | $CH_3$ | H | (300, $CDCl_3$); 0,7(3H); 0,97 u. 1,03(3H); 1,18(6H); 1,4 u.1,5 (3H); 2,16 u. 2,35(3H); 2,3(1H); 3,18(1H); 6,1(1H); 6,99-7,3 (7H); |
| 360 | $A^2$ cis | $CH_3$ | Isopropyl | H | H | (300, $CDCl_3$); 1,16-1,34(12H); 1,9 u. 2,04 (2H); 2,31(3H); 3,23(1H); 5,16(2H); 6,3(1H), 7,14-7,31(3H); |
| 361 | $A^2$ trans | $CH_3$ | Isopropyl | H | H | (300, $CDCl_3$); 1,16-1,34(12H); 1,66 u. 2,25-2,36(2H); 2,31(3H); 3,23(1H); 5,16(2H); 5,61(1H); 7,14-7,31(3H); |
| 362 | $A^5$ | $CH_3$ | Isopropyl | H | H | (300, $CDCl_3$); 1,1-1,28(6H); 1,52; 1,74-1,88 u. 2,02(2H); 2,29(3H); 3,23(1H); 3,97-4,08 u. 4,63-4,77(1H); 5,14(2H); 7,14-7,29(3H) |
| 363 | $A^5$ | $CH_3$ | Isopropenyl | H | H | (300, $CDCl_3$); 1,09-1,32(6H); 1,53; 1,77-1,9 u. ein teilweise verdecktes Signal (2H); 2,02(3H); 2,28(3H); 3,98-4,1 u. 4,62-4,75(1H); 4,83(1H); 5,14;(2H);5,21(1H);7,08-7,23(3H); |
| 364 | $A^{21}$ | F | tert.-Butyl | H | H | (200, $CDCl_3$); 0,73(3H); 0,95(3H); 1,4(9H); 2,42(1H); 4,18(1H); 5,22(2H); 6,18(2H); 6,81(2H); 7,0-7,37(3H); |
| 365 | $A^3$ | F | Isopropyl | CN | H | (300, $CDCl_3$); 1,2-1,42(12H); 1,75-2,56(2H); 3,28(1H); 5,9; 6,17 u. 6,87(1H); 6,63(1H); 7,21(1H); 7,43(2H); |

Tabelle (Fortsetzung)

| Nr. | $A^{n}$*) | $R^1$ | $R^2$ | $R^3$ | X | physik. Daten<br>$^1$H-NMR: (MHz, LM) δ [ppm]<br>Schmelzpunkt [°C], Brechungsindex |
|---|---|---|---|---|---|---|
| 366 | $A^{29}$ | Cl | Isopropyl | H | H | (200, $CDCl_3$); 0,83(3H); 1,02(3H); 1,27(6H); 2,6(1H); 3,5(1H); 4,82(1H); 5,35(2H); 6,38(1H); 7,18-7,4(3H); 7,6(1H); 7,74(1H); |
| 367 | $A^{30}$ | F | Isopropyl | H | H | (300, $CDCl_3$); 0,8(3H); 0,98(3H); 1,26(6H); 2,42(1H); 3,25(1H); 4,36(1H); 5,24(2H); 7,09(4H); 7,27(1H); 7,6(1H); |
| 368 | $A^8$ | Cl | tert.-Butyl | H | H | (300, $CDCl_3$); 0,7(3H); 1,03(3H); 1,48(9H); 2,34(1H); 3,23(1H); 5,23(2H); 7,1(2H); 7,28(4H); 7,39(1H); |
| 369 | $A^3$ | Br | Isopropyl | CN | H | (300, $CDCl_3$); 1,2-1,42(12H); 1,8; 1,88; 2,0; 2,08; 2,29 u. 2,51(2H); 3,47(1H); 5,9; 6,16 u. 6,88(1H); 6,8(1H); 7,42(2H); 7,61(1H); |
| 370 | $A^2$ | Br | Isopropyl | Isopropyl | H | (200, $CDCl_3$); 1,18-1,36(18H); 1,63-2,4(3H); 3,47(1H); 5,23(1H); 5,62 u. 6,29(1H); 7,28(3H); |
| 371 | $A^3$ | Cl | Methyl | H | H | (200, $CDCl_3$); 1,2-1,38(6H); 1,78; 1,87; 1,99; 2,07; 2,21 u. ein teilweise verdecktes Signal (2H); 2,42(3H); 5,27(2H); 5,91; 6,17 u. 6,96(1H); 7,24(3H); |
| 372 | $A^{21}$ | $OCH_3$ | Cyclohexyl | H | H. | (200, $CDCl_3$); 0,8(3H); 1,02(3H); 1,44(6H); 1,87(4H); 2,46(1H); 2,97(1H); 3,73(3H); 4,2(1H); 5,27(2H); 6,2(2H); 6,82(2H); 7,13(2H); 7,3(1H); |
| 373 | $A^2$ | Cl | Isopropyl | H | 4-Cl | (300, $CDCl_3$); 1,17-1,48(12H); 1,71, 1,94; 2,07 u. 2,28(2H); 3,94(1H); 5,2(2H); 5,63 u. 6,27(1H); 7,08-7,31(2H); |
| 374 | $A^3$ | Cl | Isopropyl | H | 4-Cl | (300, $CDCl_3$); 1,23-1,47(12H); 1,87; 2,09; 2,21 u. 2,46(2H); 3,95(1H); 5,21(2H); 6,16 u. 6,92(1H); 7,13-7,31(2H); |

Tabelle (Fortsetzung)

| Nr. | $A^{n}$ *) | $R^1$ | $R^2$ | $R^3$ | X | physik. Daten $^1$H-NMR: (MHz, LM) δ [ppm] Schmelzpunkt [°C], Brechungsindex |
|---|---|---|---|---|---|---|
| 375 | $A^2$ | Cl | n-Butyl | H | 4-Cl | (300, $CDCl_3$); 0,95(3H); 1,19-1,6(6H+4H); 1,7; 1,93; 2,08 u. 2,28 (2H); 2,94(2H); 5,19(2H); 5,62 u. 6,26(1H); 7,18(1H); 7,28(1H); |
| 376 | $A^3$ | Cl | n-Butyl | H | 4-Cl | (300, $CDCl_3$); 0,97(3H); 1,21-1,61(6H+4H); 1,77; 1,85; 1,99; 2,07; 2,2 u. 2,45(2H); 2,93(2H); 5,2(2H); 5,9; 6,17; 6,62 u. 6,92(1H); 7,18(1H); 7,28(1H) |

*) $A^n$ (n=1-27) steht für den jeweiligen Carboxylat-Rest einer im vorstehenden Text genannten Pyrethroidsäure ($A^n$-H)

Herstellung von Ausgangsprodukten gemäß der zeitgleichen Anmeldung P 38 20 896.

1-(3′-Chlor-2′-methylphenyl)cyclohexanol

Unter Stickstoffatmosphäre werden 96 g (4 mol) Mg (Späne) in 60 ml absolutem THF (Tetrahydrofuran) vorgelegt. Bei 65 °C fügt man 1 ml 1,2-Dibromethan hinzu und tropft dann eine Lösung von 644 g (4 mol) 2,6-Dichlortoluol in 1,5 l absolutem THF innerhalb von 2 1/4 Stunden hinzu. Anschließend rührt man 4 Stunden unter Rückfluß, kühlt auf Raumtemperatur und fügt unter $N_2$ 352,8 g (3,6 mol) Cyclohexanon in 250 ml absolutem THF hinzu. Nach erfolgter Umsetzung wird das Grignard-Reaktionsgemisch wie üblich wäßrig aufgearbeitet und das Lösungsmittel durch Destillation im Vakuum weitgehend entfernt. Der Rückstand wird andestilliert (30 bis 107°C/0,27 mbar). Das verbleibende Rohprodukt (676 g), welches bereits 90 % 1-(3′-Chlor-2′-methylphenyl)cyclohexanol enthält, kann mittels Säulenchromatographie (Fließmittel: Toluol) weiter gereinigt werden.

3-Cyclohexyl-2-methyl-chlorbenzol

103 g 1-(3′-Chlor-2′-methylphenyl)cyclohexen werden in 1 200 ml Ethanol gelöst und mit 5 g Pd/Kohl versetzt. Bei Raumtemperatur und 80 bar Wasserstoffdruck wird 8 Stunden lang hydriert. Man filtriert den Katalysator vom Reaktionsaustrag ab und engt die Lösung ein. Nach fraktionierter Destillation erhält man 51,8 g 3-Cyclohexyl-2-methyl-chlorbenzol.

3-(1′-Cyclohexenyl)-2-methylbenzonitril

250 g (0,6 mol) 1-(3′-Chlor-2′-methylphenyl)cyclohexen, 600 ml 1-Methyl-2-pyrrolidon und 63 g wasserfreies Kupfer(I)-cyanid werden unter Rühren 35 Stunden zum Sieden erhitzt. Das Reaktionsgemisch wird in eine Lösung von 500 ml Ethylendiamin in 1,5 l Wasser gegossen, 45 Minuten bei 50°C gerührt und mehrmals mit Toluol extrahiert. Die vereinigten organischen Phasen schüttelt man mit 10 %iger Natriumcyanid-Lösung aus, trocknet über $Na_2SO_4$ und engt ein. Nach säulenchromatographischer Reinigung über Kieselgel mit Toluol/Cyclohexan (3/7) als Elutionsmittel erhält man 117,8 g Nitril (Schmp. 54 - 57°C).

Anwendungsbeispiele

In den folgenden Beispielen dienten folgende Verbindungen als Vergleichssubstanzen A und B:

A 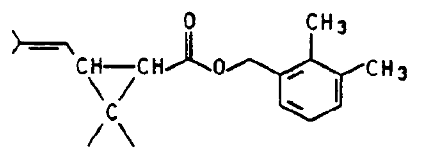 bekannt aus Pestic. Sci. 1(2), 49-52, 1970 und Pestic. Sci. 3(1), 25-28, 1972

B 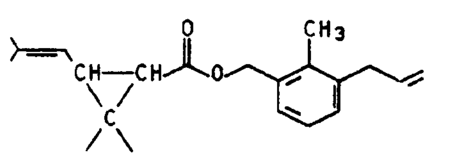 bekannt aus DE-OS 33 27 292, Beispiel 26

Die Reinheit der Substanzen wie der Vergleichsmittel lag bei >95 %.

Die Konzentrationen, bei denen die untersuchten Verbindungen eine 100 %ige bzw. 80 %ige Mortalität bzw. Hemmung bewirken, sind die jeweiligen Minimalkonzentrationen (Wirkungsschwelle). Bei jeder Konzentration wurden mindestens zwei Versuche durchgeführt und ein Mittelwert gebildet.

Kontaktwirkung auf Blatta orientalis (Schabe)

Der Boden eines 1-l-Glases wird mit der acetonischen Lösung des Wirkstoffes behandelt. Nach Verdunsten des Lösungsmittels setzt man in jedes Glas 5 adulte Schaben.

Die Mortalitätsrate wird nach 48 Stunden bestimmt.

Mit den Verbindungen der Beispiele 2 (cis/trans = 15/85), 13, 65 und 119 wird bei einer Wirkstoffkonzentration von 0,2 mg eine 100 %ige Mortalitätsrate erzielt. Bei geringeren Konzentrationen von 0,2 bis 0,04 mg zeigen die Verbindungen 2 (cis), 77, 107 und 210 Mortalitätsraten von 60 bis 100 %. Die Vergleichssubstanzen A und B sind bei einer Wirkstoffkonzentration von 1 mg unwirksam.

Kontaktwirkung auf Sitophilus granaria (Kornkäfer)

Aufgerauhte Glasplatten von 8 x 8 cm Kantenlänge werden mit der acetonischen Wirkstofflösung behandelt.

Nach Verdunsten des Lösungsmittels belegt man die Platten mit 100 Kornkäfern und deckt mit einem Uhrglas (∅ 6 cm) ab. Nach 4 Stunden werden die Käfer in unbehandelte Gefäße überführt. Die Mortalitätsrate wird nach 24 Stunden ermittelt. Dabei wird festgestellt, wieviele Käfer in der Lage waren, nach diesem Zeitpunkt innerhalb von 60 Minuten ein unbehandeltes Pappschälchen (∅ 40 mm, Höhe 10 mm) zu verlassen.

Mit den Verbindungen der Beispiele 1, 2 (cis), 2 (cis,trans = 15/85) 5, 107, 173, 193, 204, 210 und 268 werden bei Wirkstoffkonzentrationen zwischen 0,04 bis 0,4 mg Mortalitätsraten von 0 bis 100 % erzielt. Die Vergleichssubstanzen A und B sind bei einer Wirkstoffkonzentration von 1 mg unwirksam.

Kontaktwirkung auf Aedes aegypti (Gelbfiebermücke)

200 ml Leitungswasser in 250 ml Plastikbechern werden mit der Wirkstoffaufbereitung versetzt und darauf mit 20 bis 30 Moskito-Larven im 3. bis 4. Larvenstadium besetzt.

Die Versuchstemperatur beträgt 25°C. Nach 24 Stunden wird die Wirkung ermittelt.

Hierbei erzielen die Wirkstoffe der Beispiele 2 (cis/trans = 15/85), 2 (cis), 5, 107, 113, 119, 173, 179 ca. 80 % Mortalität bei Anwendungskonzentrationen von 0,02 ppm, 0,01 ppm und weniger. Die Vergleichssubstanzen A und B sind bei Konzentrationen von 0,1 ppm unwirksam.

Dauerkontakt auf Musca domestica (Stubenfliegen)

Beide Teile einer Petrischale von 10 cm Durchmesser werden mit insgesamt 2 ml der acetonischen Wirkstofflösung ausgekleidet. Nach Verdunsten des Lösungsmittels (ca. 30 Minuten) bringt man je 10 Fliegen in die Schalen. Die Mortalität wird nach 4 Stunden festgestellt.

Hierbei erzielen die Wirkstoffe der Beispiele 1, 2 (cis/trans = 15/85), 5, 107, 113, 119, 173, 179 und 210 eine Mortalitätsrate von ca. 80 bis 100 % bei Anwendungskonzentrationen im Bereich von 0,004 bis 0,001 mg. Die Vergleichssubstanz A führt mit 0,1 mg zu einer Mortalitätsrate von ca. 80 % während die Vergleichssubstanz B bei dieser Konzentration 100 % Mortalität erzielte.

Kontaktwirkung auf Ornithodorus moubata (Zecke)

Verwendet werden junge Zecken, die eine Blutmahlzeit hinter sich haben (Durchmesser 1,5 bis 2 mm). Diese Tiere nimmt man mit Hilfe eines Saugrohres einzeln auf, wobei eine starke Lichtquelle die aktiven Tiere aus den Häutungsresten treibt.

Je 5 Zecken werden in einem flüssigkeitsdurchlässigen Beutel für 5 Sekunden in die wäßrige Wirkstoffaufbereitung getaucht. Die Beutel hängt man danach frei auf und ermittelt nach 48 Stunden die Wirkung, indem man die Beutel auf eine Wärmeplatte legt und die lebenden Tiere in ihrer Aktivität leicht erkennt.

Mit den Verbindungen der Beispiele 18, 193, 210 und 268 werden bei Wirkstoffkonzentrationen von 0,4 bis 4 ppm 60 bis 80 % Mortalität erzielt. Eine Mortalitätsrate von 100 % zeigen die Verbindungen 5 und 13 bei Konzentrationen von 5 und 10 ppm. Erst bei einer viel höheren Konzentration von 400 ppm kann mit der Vergleichsssubstanz A 100 % Mortalität erreicht werden, während die Vergleichssubstanzen B selbst bei 1000 ppm unwirksam ist.

Plutella maculipennis (Kohlschaben-Raupen), Fraßwirkung

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt.

Nach 48 Stunden beurteilt man die Wirkung.

Mit den Verbindungen der Beispiele 1, 5, 18, 107, 119, 179, 204 und 268 werden Mortalitätsraten von 80

bis 100 % bei Anwendungskonzentrationen von 0,4 bis 20 ppm erzielt. Bei Konzentrationen von 40 ppm zeigten die Verbindungen 13, 60, 65 und 193 Mortalitätsraten von 60 bis 100 %. Die Vergleichssubstanz A führt erst bei 400 ppm zu 100 % Mortalität, mit Vergleichssubstanz B wird bei 40 ppm 100 % Mortalität erzielt.

Kontaktwirkung auf Dysdercus intermedius (Baumwollwanze)

Petrischalen von 10 cm Durchmesser werden mit 1 ml der acetonischen Wirkstofflösung ausgekleidet. Nach Verdunsten des Lösungsmittels besetzt man die Schalen mit je 20 Larven des vorletzten Stadiums und registriert die Wirkung nach 24 Stunden.

Hierbei erzielten die Verbindungen der Beispiele 1, 2 (cis), 2 (cis/trans = 15/85), 107, 113, 179 und 204 eine 100 %ige Mortalitätsrate bei Wirkstoffkonzentrationen im Bereich von 0,1 bis 1 ppm. Die Vergleichssubstanzen A und B sind bei Konzentrationen von 10 ppm unwirksam.

Kontaktwirkung auf Prodenia litura (Ägyptischer Baumwollwurm)

Der Boden von Glaspetrischalen (∅ 10 cm) wird mit der acetonischen Wirkstofflösung behandelt. Nach dem Verdunsten des Lösungsmittels besetzt man die Schalen mit 5 Raupen des 3. Stadiums und verschließt sie. Nach 4 Stunden wird die Mortalität in % festgestellt.

Hierbei erzielen die Verbindungen der Beispiele 2 (cis/trans = 15/85), 5, 107, 113, 119, 179, 204 und 268 ca. 80 % Mortalität bei Anwendungskonzentrationen im Bereich von 0,01 bis 0,001 mg. Das cis-Isomer 2 zeigt 100 % Mortalität bei einer Konzentration von 0,001 mg. Mit den Vergleichssubstanzen werden 100 % Mortalität bei Konzentrationen von 0,02 mg (A) bzw. 0,04 mg (B) erzielt.

## Patentansprüche

1. Benzylester der allgemeinen Formel I

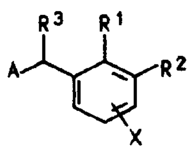

(I)

in der

$R^1$ Methyl, Ethyl, Halogen, Methoxy oder Ethoxy,

$R^2$ Alkyl, Alkenyl, Cycloalkyl, Cycloalkenyl, Bicycloalkyl, Bicycloalkenyl, $C_1$-$C_5$-alkylsubstituiertes Cycloalkyl, $C_1$-$C_5$-alkylsubstituiertes Cycloalkenyl, $C_1$-$C_5$-alkylsubstituiertes Bicycloalkyl oder $C_1$-$C_5$-alkylsubstituiertes Bicycloalkenyl,

$R^3$ Wasserstoff, Cyano, $C_2$-$C_4$-Alkinyl, $C_2$-$C_4$-Alkenyl, oder $C_1$-$C_4$-Alkyl,

A den Carboxylat-Rest einer bei Pyrethroiden typischen Säurekomponente und

X Wasserstoff oder Halogen

bedeuten, mit der Maßgabe, daß $R^2$ nicht den Rest $CH_2$-CH=CH-B bedeutet, wenn B für Wasserstoff, Alkyl oder Alkenyl steht und zugleich $R^1$ die Bedeutung Methyl oder Halogen hat, sowie ferner mit der Maßgabe, daß $R^2$ nicht Methyl bedeutet, wenn zugleich $R^1$ für Methyl steht.

2. Verbindungen nach Anspruch 1, dadurch gekennzeichnet, daß $R^2$, $R^3$ und A die in Anspruch 1 angegebene Bedeutung haben, X für Wasserstoff und $R^1$ für Methyl, Chlor, Fluor oder Brom steht.

3. Verbindungen gemäß Anspruch 1, dadurch gekennzeichnet, daß X und $R^3$ für Wasserstoff stehen.

4. Verbindungen gemäß den Ansprüchen 1 bis 3, dadurch gekennzeichnet, daß A für die Carboxylat-Reste II, III oder IV

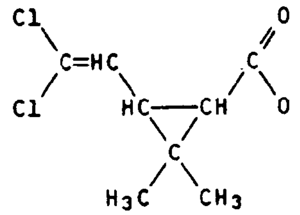

(II)

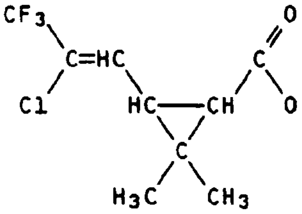

(III)

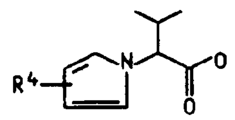

(IV)

steht, wobei $R^4$ die Bedeutung Wasserstoff, $C_1$-$C_4$-Alkyl oder $C_1$-$C_4$-Halogenalkyl hat.

5. Verbindungen gemäß den Ansprüchen 1 bis 4, dadurch gekennzeichnet, daß $R^2$ Isopropyl, Isopropenyl oder sec.-Butyl bedeutet.

6. Verfahren zur Herstellung von Verbindungen nach den Ansprüchen 1 bis 5, dadurch gekennzeichnet, daß man bei Pyrethroiden typischen Säuren der allgemeinen Formel A-H oder Säurederivate hiervon mit Verbindungen der allgemeinen Formel V oder Derivaten dieser Verbindungen,

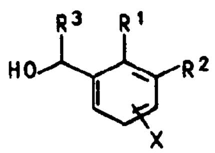

(V),

wobei A, $R^1$ bis $R^3$ und X jeweils die in den Ansprüchen 1 bis 5 angegebene Bedeutung haben, bei Temperaturen von -20°C bis +150°C gegebenenfalls in Gegenwart eines Lösungsmittels, eines Katalysators, eines Säurebindemittels, eines Alkali- oder Erdalkalicyanids umsetzt.

7. Schädlingsbekämpfungsmittel, enthaltend feste oder flüssige Trägerstoffe und mindestens eine Verbindung gemäß den Ansprüchen 1 bis 5.

8. Schädlingsbekämpfungsmittel nach Anspruch 7, dadurch gekennzeichnet, daß es 0,01 bis 95 Gew.% eines Benzylesters der Formel I enthält.

9. Verfahren zur Bekämpfung von Schädlingen, dadurch gekennzeichnet, daß man Benzylester gemäß den Ansprüchen 1 bis 5 auf die Schädlinge bzw. deren Lebensraum einwirken läßt.

10. Verwendung von Benzylestern gemäß den Ansprüchen 1 bis 5 zur Bekämpfung von Schädlingen.

## Claims

1. A benzyl ester of the formula

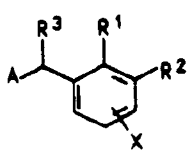

(I)

where $R^1$ is methyl, ethyl, halogen, methoxy or ethoxy, $R^2$ is alkyl, alkenyl, cycloalkyl, cycloalkenyl, bicycloalkyl,bicycloalkenyl,$C_1$-$C_5$-alkyl-substituted cycloalkyl, $C_1$-$C_5$-alkyl-substituted cycloalkenyl, $C_1$-$C_5$-alkyl-substituted bicycloalkyl or $C_1$-$C_5$-alkyl-substituted bicycloalkenyl, $R^3$ is hydrogen, cyano, $C_2$-$C_4$-alkynyl, $C_2$-$C_4$-alkenyl or $C_1$-$C_4$-alkyl, A is the carboxylate radical of an acid component typical for pyrethroids and X is hydrogen or halogen, with the proviso that $R^2$ is not $CH_2$-CH=CH-B when B is hydrogen, alkyl or alkenyl and at the same time $R^1$ is methyl or halogen, and furthermore with the proviso that $R^2$ is not methyl when at the same time $R^1$ is methyl.

2. A compound as claimed in claim 1, wherein $R^2$, $R^3$ and A have the meanings given in claim 1, X is hydrogen and $R^1$ is methyl, chlorine, fluorine or bromine.

3. A compound as claimed in claim 1, wherein X and $R^3$ are each hydrogen.

4. A compound as claimed in claims 1 to 3, wherein A is a carboxylate radical II, III or IV

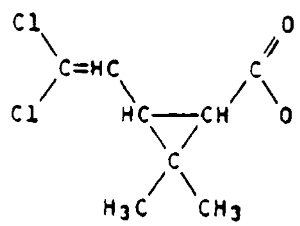

(II)

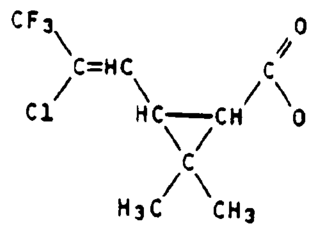

(III)

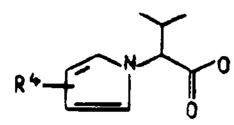

(IV)

where $R^4$ is hydrogen, $C_1$-$C_4$-alkyl or $C_1$-$C_4$-haloalkyl.

5. A compound as claimed in claims 1 to 4, wherein $R^2$ is isopropyl, isopropenyl or sec-butyl.

6. A process for the preparation of compounds as claimed in claims 1 to 5, wherein an acid, typical for pyrethroids, of the formula A-H, or an acid derivative thereof, is reacted with a compound of the formula V, or a derivative thereof,

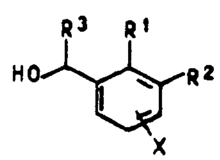

(V)

where A, $R^1$ to $R^3$ and X have the meanings given in claims 1 to 5, at from -20 to +150°C and in the presence or absence of a solvent, a catalyst, an acid binder and an alkali metal or alkaline earth metal cyanide.

7. A pesticide containing solid or liquid carriers and at least one compound as claimed in claims 1 to 5.

8. A pesticide as claimed in claim 7, which contains from 0.01 to 95% by weight of a benzyl ester of the formula I.

9. A process for combating pests, wherein a benzyl ester as claimed in claims 1 to 5 is allowed to act on the pests or their habitat.

10. The use of a benzyl ester as claimed in claims 1 to 5 for combating pests.

## Revendications

1. Esters benzyliques de formule générale I

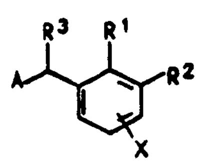

(I)

dans laquelle

$R^1$ représente un groupe méthyle, éthyle, un halogène, un groupe méthoxy ou éthoxy,

$R^2$ représente un groupe alkyle, alcényle, cycloalkyle, cycloalcényle, bicycloalkyle, bicycloalcényle, cycloalkyle à substituants alkyle en C1-C5, cycloalcényle à substituants alkyle en C1-C5, bicycloalkyle à substituants alkyle en C1-C5 ou bicycloalcényle à substituants alkyle en C1-C5

$R^3$ représente l'hydrogène, un groupe cyano, alcynyle en C2-C4, alcényle en C2-C4 ou alkyle en C1-C4

A représente le radical carboxylate d'un composant acide typique des pyréthroïdes et

X représente l'hydrogène ou un halogène,

sous réserve que $R^2$ ne peut représenter le groupe $CH^2$-CH=CH-B lorsque B représente l'hydrogène, un groupe alkyle ou alcényle et que, simultanément, $R^1$ représente un groupe méthyle ou un halogène, et sous réserve que $R^2$ ne peut représenter un groupe méthyle lorsque, simultanément, $R^1$ représente un groupe méthyle.

2. Composés selon la revendication 1, caractérisés en ce que $R^2$, $R^3$ et A ont les significations indiquées dans la revendication 1, X représente l'hydrogène et $R^1$ un groupe méthyle, le chlore, le fluor ou le brome.

3. Composés selon la revendication 1 à 3, caractérisés en ce que X et $R^3$ représentent l'hydrogène.

4. Composés selon les revendications 1 à 3, caractérisés en ce que A représente les radicaux carboxylates II, III ou IV

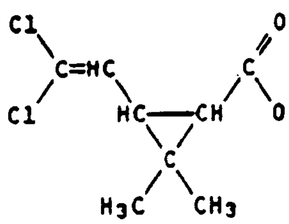

(II)

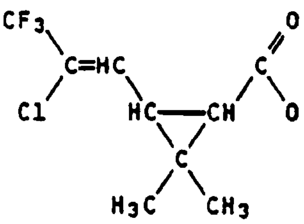

(III)

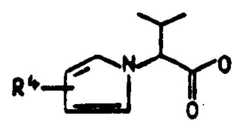

(IV)

dans lesquels $R^4$ représente l'hydrogène, un groupe alkyle en C1-C4 ou halogènoalkyle en C1-C4.

5. Composés selon les revendications 1 à 4, caractérisés en ce que $R^2$ représente un groupe isopropyle, isopropényle ou sec-butyle.

6. Procédé de préparation des composés selon les revendications 1 à 5, caractérisé en ce que l'on fait réagir des acides de formule générale A-H typiques des pyréthroïdes ou leurs dérivés avec des composés de formule générale V ou leurs dérivés

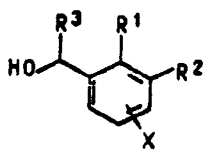

(V),

dans lesquels A, $R^1$ à $R^3$ et X ont les significations indiquées dans les revendications 1à 5, à des températures de -20 à +150 degrés C, éventuellement en présence d'un solvant, d'un catalyseur, d'un accepteur d'acide, d'un cyanure alcalin ou alcalino-terreux.

7. Produit pesticide contenant des véhicules solides ou liquides et au moins un composé selon les revendications 1 à 5.

8. Produit pesticide de la revendication 7, caractérisé en ce qu'il contient de 0,01 à 95 % en poids d'un ester benzylique de formule I.

9. Procédé pour combattre les parasites, caractérisé en ce que l'on fait agir des esters benzyliques selon les revendications 1 à 5 sur les parasites ou leur habitat.

10. Utilisation des esters benzyliques selon les revendications 1 à 5 pour la lutte contre les parasites.